# EUROPEAN PATENT SPECIFICATION

(11) **EP 4 441 041 B1**
(45) Date of publication and mention of the grant of the patent: **17.09.2025**
(21) Application number: 22822902.7
(22) Date of filing: 25.11.2022
(51) Int. Cl.: C07D 401/14, C07D 403/06, A01N 43/54, A01N 43/56

(54) **HERBICIDAL PYRAZOLE PYRIMIDINE COMPOUNDS**
HERBIZIDE PYRAZOLPYRIMIDINVERBINDUNGEN
COMPOSÉS DE PYRAZOLE PYRIMIDINE HERBICIDES

(30) Priority: 03.12.2021 GB 202117474
(43) Date of publication of application: 09.10.2024
(73) Proprietor: Syngenta Crop Protection AG, 4058 Basel (CH)
(72) Inventor: DALE, Suzanna Jane, Bracknell Berkshire RG42 6EY (GB); ELVES, Philip Michael, Bracknell Berkshire RG42 6EY (GB); MORRIS, James Alan, Bracknell Berkshire RG42 6EY (GB); WATKIN, Samuel Vaughan, Bracknell Berkshire RG42 6EY (GB)
(74) Representative: SYNGENTA IP
(86) International application number: PCT/EP2022/083333
(87) International publication number: WO 2023/099354

(56) References cited:
- WO-A1-2021/204706
- WO-A1-2022/101270
- CN-B- 105 037 342

## Description

The present invention relates to novel herbicidal compounds, to processes for their preparation, to herbicidal compositions which comprise the novel compounds, and to their use for controlling weeds, in particular in crops of useful plants, or for inhibiting plant growth.

Herbicidal pyrazole compounds are disclosed in, for example, CN105037342. The present invention relates to novel herbicidal pyrazole compounds. Thus, according to the present invention there is provided a compound of Formula (I): 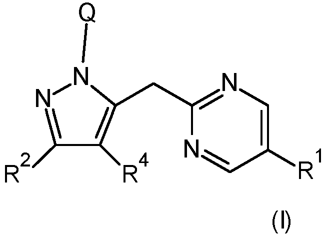
or an agronomically acceptable salt thereof,
wherein
Q is selected from the group consisting of phenyl, a C-linked 6-membered heteroaryl, Q-A and Q-B 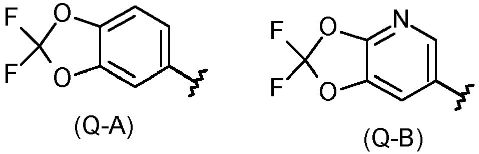
   wherein said phenyl or 6-membered heteroaryl are optionally substituted by one or more independent R³ substituents;
   R¹ is independently selected from the group consisting of halogen, -CN, NO₂, C₁-C₄alkyl, C₁-C₄haloalkyl, C₃-C₆cycloalkyl, C₂-C₄alkenyl, C₂-C₄alkynyl, - S(O)ₚC₁-C₄alkyl, C₁-C₄alkoxy-, -C(O)C₁-C₄alkyl, -C(O)OC₁-C₄alkyl, C₁-C₄haloalkoxy and C₁-C₄alkoxyC₁-C₃alkyl-;
   R² is selected from the group consisting of halogen, -CN, C₁-C₂alkyl and C₁-C₂haloalkyl;
   R³ is selected from the group consisting of halogen, C₁-C₄ alkyl, C₁-C₄ haloalkyl, C₁-C₄ alkoxy, C₁-C₄ haloalkoxy, -CN, NO₂, C₂-C₄alkenyl, C₂-C₄alkynyl, -S(O)ₚC₁-C₄alkyl;
   R⁴ is selected from the group consisting of hydrogen, halogen, -CN, C₁-C₂alkyl and C₁-C₂haloalkyl; and
   p = 0, 1 or 2.

C₁-C₄alkyl- includes, for example, methyl (Me, CH₃), ethyl (Et, C₂H₅), *n*-propyl (n-Pr), isopropyl (i-Pr), n-butyl (n-Bu), isobutyl (i-Bu), sec-butyl and *tert*-butyl (*t*-Bu). C₁-C₂alkyl is methyl (Me, CH₃) or ethyl (Et, C₂H₅).

C₂-C₄alkenyl- includes, for example, -CH=CH₂ (vinyl) and -CH₂-CH=CH₂ (allyl).

C₂-C₄alkynyl- refers to a straight or branched hydrocarbon chain radical group consisting solely of carbon and hydrogen atoms, containing at least one triple bond, having from two to four carbon atoms, and which is attached to the rest of the molecule by a single bond. Examples of C₂₋C₄alkynyl include, but are not limited to, prop-1-ynyl, propargyl (prop-2-ynyl), and but-1-ynyl.

Halogen (or halo) includes, for example, fluorine, chlorine, bromine or iodine. The same correspondingly applies to halogen in the context of other definitions, such as haloalkyl.

C₁-C₄haloalkyl- includes, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoropropyl and 2,2,2-trichloroethyl and heptafluoro-n-propyl. C₁-C₂haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, or 1,1-difluoro-2,2,2-trichloroethyl.

C₁-C₄alkoxy includes methoxy and ethoxy.

C₁-C₄haloalkoxy- includes, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₃-C₆cycloalkyl includes cyclopropyl, cyclopentyl and cyclohexyl.

C₁-C₄alkyl-S- (alkylthio) includes, for example, methylthio, ethylthio, propylthio, isopropylthio, n-butylthio, isobutylthio, sec-butylthio or tert-butylthio, preferably methylthio or ethylthio.

C₁-C₄alkyl-S(O)- (alkylsulfinyl) includes, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₄alkyl-S(O)₂- (alkylsulfonyl) includes, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In a preferred embodiment of the present invention, there is provided a compound of Formula (I) wherein Q is selected from the group consisting of:

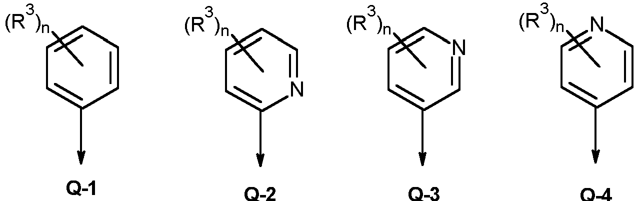

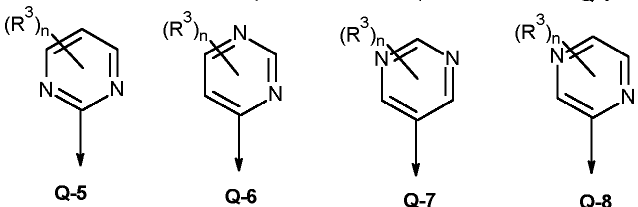

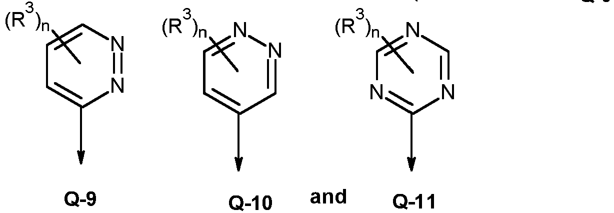

In another embodiment of the present invention, Q is selected from the group consisting of Q-1, Q-3 and Q-4.

In a more preferred embodiment of the present invention, Q is Q-1 or Q-3.

In a more preferred embodiment of the present invention, Q is Q-1.

In another preferred embodiment of the present invention, Q is Q-3.

In one embodiment of the present invention, R³ is selected from the group consisting of halogen, C₁-C₄ alkyl (e.g methyl), C₁-C₄ haloalkyl (e.g -CF₃ or -CHF₂), - CN and -S(O)ₚC₁-C₄alkyl (e.g -SO₂methyl). More preferably R³ is halogen (preferably fluoro or chloro) or C₁-C₄ haloalkyl (e.g -CF₃ or -CHF₂) e.g halogen or -CF₃.

In another embodiment of the present invention n is 0, 1 or 2, preferably 1 or 2. In one embodiment n is 1. In another embodiment n is 2. It appears that compounds wherein n = 2 exhibit improved selectivity (good weed control with little, if any, crop damage).

In a more preferred embodiment, Q is Q-1, R³ is halogen or C₁-C₄ haloalkyl and n is 1 (wherein in a preferred embodiment Q is 4-CF₃-phenyl-).

In another preferred embodiment of the present invention Q is Q-1, R³ is halogen and n is 2 (wherein in a preferred embodiment Q is 3,4-dihalophenyl- (e.g 3-F,4-Cl-phenyl-, 3-Cl,4-F-phenyl-, 3,4-diclorophenyl- or 3,4diflurophenyl-).

In another preferred embodiment of the present invention Q is Q-3, R³ is halogen and n is 2 (wherein in a preferred embodiment Q is 6,5-dihalo-3-pyridyl-phenyl- (e.g 6-chloro-5-fluoro-3-pyridyl).

In another embodiment of the invention Q is Q-A or QB:

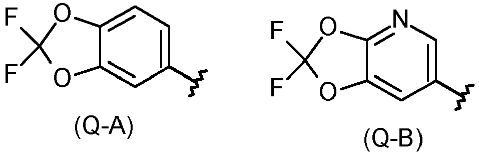

In another embodiment of the present invention, R¹ is selected from the group consisting of halogen, CN, C₁-C₄alkyl, C₁-C₄haloalkyl, C₁-C₄alkoxy and C₁-C₄haloalkoxy. In a more preferred embodiment, R¹ is selected from the group consisting of methyl, fluoro, chloro, bromo and methoxy-. In an even more preferred embodiment, R¹ is chloro.

In another embodiment of the present invention, R² is halogen (e.g chloro), - CN or C₁-C₂haloalkyl. In a preferred embodiment R² is -CF₃ or -CF₂H.

In another embodiment of the present invention, R⁴ is hydrogen.

Compounds of Formula (I) may contain asymmetric centres and may be present as a single enantiomer, pairs of enantiomers in any proportion or, where more than one asymmetric centre are present, contain diastereoisomers in all possible ratios. Typically, one of the enantiomers has enhanced biological activity compared to the other possibilities.

The present invention also provides agronomically acceptable salts of compounds of Formula (I). Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred.

The compounds of Formula (I) according to the invention can be used as herbicides by themselves, but they are generally formulated into herbicidal compositions using formulation adjuvants, such as carriers, solvents and surface-active agents (SAA). Thus, the present invention further provides a herbicidal composition comprising a herbicidal compound according to any one of the previous claims and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface-active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkyl naphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates.

Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).
The compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron,fomesafen, flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester,4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]-imidazolidine-2-one, 5-ethoxy-4-hydroxy-1-methyl-3-[4-(trifluoromethyl)-2-pyridyl]-imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate, prop-2-ynyl 4-amino-3-chloro-5-fluoro-6-(7-fluoro-1H-indol-6-yl)pyridine-2-carboxylate and cyanomethyl 4-amino-3-chloro-5-fluoro-6-(7-fluoro-1H-indol-6-yl)pyridine-2-carboxylate), 3-ethyl-sulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonyl-methyl)-N-(5-methyl-1,3,4-oxadiazol-2-yl)-5-(trifluoromethyl)-[1,2,4]triazolo[4,3-a]-pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl-2-[[3-[[3-chloro-5-fluoro-6-[3-methyl-2,6-dioxo-4-(trifluoromethyl)pyrimidin-1-yl]-2-pyridyl]oxy]acetate,6-chloro-4-(2,7-dimethyl-1-naphthyl)-5-hydroxy-2-methyl-pyridazin-3-one, tetrahydrofuran-2-ylmethyl(2R)-2-[(4-amino-3,5-dichloro-6-fluoro-2-pyridyl)oxy]-propanoate, (2R)-2-[(4-amino-3,5-dichloro-6-fluoro-2-pyridyl)oxy]propanoic acid, tetrahydrofuran-2-ylmethyl2-[(4-amino-3,5-dichloro-6-fluoro-2-pyridyl)oxy]propanoate, 2-[(4-amino-3,5-dichloro-6-fluoro-2-pyridyl)oxy]propanoic acid, 2-fluoro-N-(5-methyl-1,3,4-oxadiazol-2-yl)-3-[(R)-propylsulfinyl]-4-(trifluoromethyl)benzamide, 2-fluoro-N-(5-methyl-1,3,4-oxadiazol-2-yl)-3-propylsulfinyl-4-(trifluoromethyl)benzamide, (2-fluorophenyl)methyl6-amino-5-chloro-2-(4-chloro-2-fluoro-3-methoxyphenyl)-pyrimidine-4-carboxylate, 6-amino-5-chloro-2-(4-chloro-2-fluoro-3-methoxy-phenyl)-pyrimidine-4-carboxylic acid, 3-(3-chlorophenyl)-6-(5-hydroxy-1,3-dimethyl-pyrazole-4-carbonyl)-1,5-dimethyl-quinazoline-2,4-dione and [4-[3-(3-chlorophenyl)-1,5-dimethyl-2,4-dioxo-quinazoline-6-carbonyl]-2,5-dimethyl-pyrazol-3-yl]N,N-diethylcarbamate.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or metcamifen.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus said method comprising application to the locus of a weed controlling amount of a composition comprising a compound of Formula (I). Moreover, the present invention may further provide a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. It is noted that the compounds of the present invention show a much-improved selectivity compared to know, structurally similar compounds. Generally the plants to be controlled are unwanted plants (weeds). 'Locus' means the area in which the plants are growing or will grow. The application may be applied to the locus pre-emergence and/or postemergence of the crop plant. Some crop plants may be inherently tolerant to herbicidal effects of compounds of Formula (I). Preferred crop plants include maize, wheat, barley soybean and rice.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to other herbicides or classes of herbicides (e.g. ALS-, GS-, EPSPS-, PPO-, HPPD-, -PDS and ACCase-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}. The compounds of the present invention can also be used in conjunction with crops that are tolerant to SDPS-inhibiting herbicides, such as those taught in WO2020/236790.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are Knockout^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compositions can be used to control unwanted plants (collectively, 'weeds'). The weeds to be controlled may be both monocotyledonous species, for example *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

In a further aspect of the present invention there is provided the use of a compound of Formula (I) as defined herein as a herbicide.

### Processes for preparation of compounds of Formula (I)

Processes for preparation of compounds, e.g. a compound of Formula (I) (which optionally can be an agrochemically acceptable salt thereof), are now described, and form further aspects of the present invention.

As shown in Scheme 1, a compound of Formula (I) can be prepared via decarboxylation of compounds of Formula (2) by heating at 110°C under acidic conditions in a suitable solvent such as ethanol.

Compounds of Formula (2) are prepared using a nucleophilic aromatic substitution reaction of compounds of Formula (4) (where LG is represents a suitable leaving group such as halogen or SO₂Me) by heating in a suitable solvent, such as sulfolane in the presence of a base such as sodium t-butoxide with a compound of Formula (3). The reaction is typically conducted at 40°C.

Conditions for the formation of pyrazole compounds of Formula (3) are documented in the literature via the condensation of diketones with an arylhydrazines (as documented in Tetrahedron (2013), 69(16), 3459-3464).

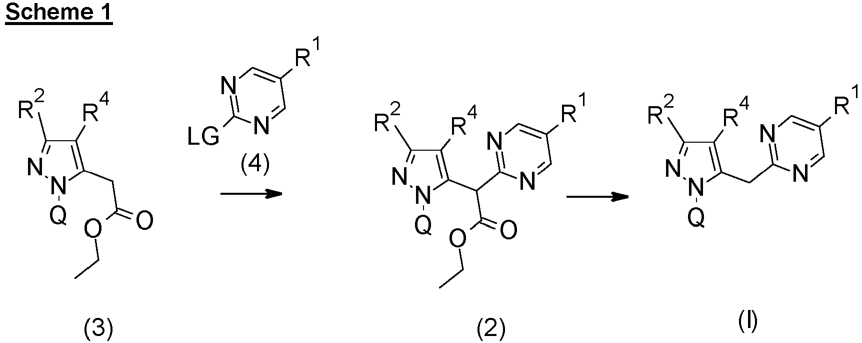

Alternatively compounds of formula I can be prepared by following Scheme 2.

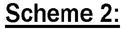

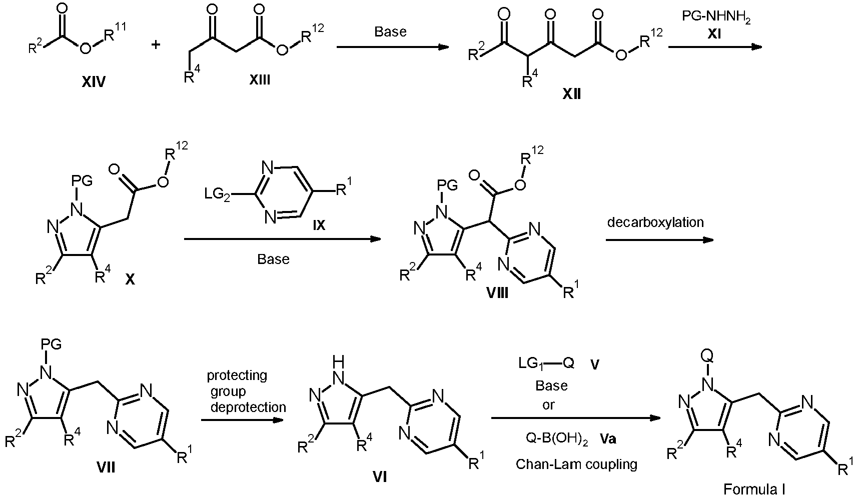

In scheme 2 compounds of formula I can be prepared by reacting compounds of formula **VI**, with reagents of the formula **V**, wherein LG₁ is a halogen, preferably iodine, bromine or chlorine (or a pseudo-halogen leaving group, such as a (halo)alkyl or phenyl sulfonate ester, e.g. triflate), in the presence of a base, such as sodium hydride or an alkali earth metal hydride, carbonate (e.g. sodium carbonate, potassium carbonate or cesium carbonate) or hydroxide, optionally in the presence of potassium iodide in an inert solvent such as tetrahydrofuran, dioxane, water, N,N-dimethylformamide DMF, N,N-dimethylacetamide, sulfolane or acetonitrile and the like, at temperatures between 0 and 120°C, by procedures well known to those skilled in the art.

Alternatively compounds of formula I can be prepared by reacting compounds of formula **VI** with compounds of formula V, wherein LG₁ is a halogen, preferably iodine, bromine or chlorine (or a pseudo-halogen leaving group, such as a (halo)alkyl or phenyl sulfonate ester, e.g. triflate) in the presence of a metal catalyst such as copper based catalyst for eg Cul or tetrakis(acetonitrile)copper(I) tetrafluoroborate optionally in the presence of a ligand such as trans-1,2-bis(methylamino)cyclohexane or its salt (for eg methanesulfonate salt) or 8-hydroxyquinoline amongst similar other ligands. The reaction can be carried out in the presence of a base such as potassium carbonate, cesium carbonate, triethylamine or pyridine and similar others and in the presence of a solvent such as acetonitrile, 1,4-dioxane or pyridine and optionally under microwave irradiation at temperature in the range of room temperature and 200 °C.

Alternatively compounds of formula I can be prepared by reacting compounds of formula **VI** and compounds of formula **Va** under Chan Lam cross-coupling reaction conditions. Such reactions are carried out in the presence of copper-based catalyst such as copper acetate or copper iodide or copper bromide and similar others and in the presence of a base such as pyridine or 2,6-lutidine and similar others. The reaction can be carried out in the presence of a solvent such as dichloromethane, toluene, acetonitrile and in the presence of air or oxygen and at temperature in the range of room temperature and 200 °C.

Compounds of formula **VI** can be prepared by protecting group deprotection reaction from compounds of formula **VII**, wherein PG is an amino-protecting group for example acetyl, trimethylsilylethoxymethyl (SEM), tert-butyloxycarbonyl, benzyl, p-methoxybenzyl (PMB) amongst others amino protecting groups. Such reactions are well known to those skilled in the art and can be carried out for example under base catalyzed such as using sodium hydroxide for the deprotection of acetyl group or under acid catalyzed such as hydrochloric acid or 2,2,2-trifluoroacetic acid for the deprotection of trimethylsilylethoxymethyl (SEM), tert-butyloxycarbonyl or p-methoxybenzyl (PMB) group.

Compounds of formula **VII** can be prepared from compounds of formula **VIII,** wherein R¹² is C₁-C₄alkyl or phenyl *via* decarboxylation reaction. The reaction can be carried out using base such as alkaline earth metal hydroxide or alkali metal hydroxide like sodium hydroxide or in the presence of acid such as aqueous hydrochloric acid, sulfuric acid amongst others. The reaction is generally carried out in the presence of a solvent such as water, ethanol, methanol, tetrahydrofuran or dioxane or combination of two or more solvent and at temperature in the range of room temperature to boiling point of solvent.

Compounds of formula **VIII,** wherein R¹² is C₁-C₄alkyl or phenyl can be prepared by reacting compounds of formula **X,** with reagents of the formula **IX,** wherein LG₂ is a halogen, (or a pseudo-halogen leaving group, such as a (halo)alkyl or phenyl sulfonate ester, e.g. triflate), in the presence of a base, such as sodium tert-butoxide, sodium hydride or an alkali earth metal hydride, carbonate (e.g. sodium carbonate, potassium carbonate or cesium carbonate) or hydroxide, or phosphate such as potassium phosphate optionally in the presence of potassium iodide in an inert solvent such as tetrahydrofuran, dioxane, water, N,N-dimethylformamide DMF, N,N-dimethylacetamide, dimethyl sulfoxide, sulfolane or acetonitrile and the like, at temperatures between 0 and 200°C, by procedures well known to those skilled in the art.

Compounds of formula **X** can be prepared by the condensation reaction of compounds of formula **XII** with compounds of formula **XI** (or its hydrochloric acid salt or trifluoroacetic acid salt), wherein PG is an amino-protecting group for example acetyl, trimethylsilylethoxymethyl (SEM), tert-butyloxycarbonyl, benzyl, p-methoxybenzyl (PMB) amongst others amino protecting groups. Such reactions are well known in the literature and can be carried out optionally in the presence of an acid catalyst such as acetic acid.

Compounds of formula **XII** can be prepared by reacting compounds of formula **XIV,** wherein R¹¹ is C₁-C₄alkyl or phenyl with compounds of formula **XIII** in the presence of a base. Such reactions are known by the name of Claisen condensation reaction and well known to those skilled in the art. Reaction can be carried out using base such as lithium diisopropylamide, lithium tetramethylpiperidide, sodium ethoxide, sodium hydride amongst other bases in the presence of solvent such as tetrahydrofuran, ethanol, methanol and at temperature in the range of -80 °C to boiling point of solvent.

Alternatively compounds of formula I can be prepared by following Scheme 3.

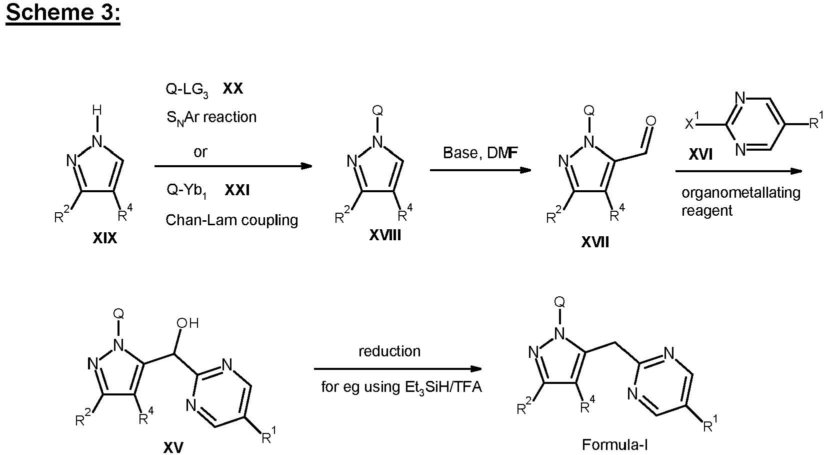

In Scheme 3 compounds of formula-I are prepared from compounds of formula XV *via* reduction of alcohol. Reduction of such alcohols are well described in literature and can be carried out using reducing agent such as LiAlH₄, DIBAL-H, or using triphenyl phosphine in the presence of iodine and imidazole or using triethyl silane in the presence of trifluoroacetic acid. Compounds of formula **XV** can be prepared by reacting compounds of formula **XVI,** wherein X¹ is a halogen preferably bromine or iodine with an organometallic reagent such as BuLi or isopropylmagnesium chloride/LiCl complex amongst other metallating reagents to form an intermediate **XVIa,** wherein M(Ln)^{P} is a corresponding metal from the organometallic reagent such as lithium or magnesium and (Ln)^{p} is its optionally substituted group like chloro and then subsequently reacting with compounds of formula **XVII.**

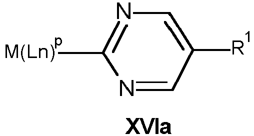

Compounds of formula **XVII** can be prepared by reacting compounds of formula **XVIII** with strong bases such as butyl lithium, lithium diisopropylamide and then reacting with DMF. The reaction is generally carried out in the presence of a solvent such as tetrahydrofuran, toluene, heptane and at temperature between -80 °C to boiling point of solvent. Such reactions are well known and described in literature. Compounds of formula **XVIII** can be prepared by reacting compounds of formula **XIX** and compounds of formula **XX,** wherein LG₃ is a leaving group like halogen (or a pseudo-halogen leaving group, such as a (halo)alkyl or phenyl sulfonate ester, e.g. triflate) in the presence of a base, such as sodium tert-butoxide, sodium hydride or an alkali earth metal hydride, carbonate (e.g. sodium carbonate, potassium carbonate or cesium carbonate) or hydroxide, or phosphate such as potassium phosphate optionally in the presence of potassium iodide in an inert solvent such as tetrahydrofuran, dioxane, water, N,N-dimethylformamide DMF, N,N-dimethylacetamide, dimethyl sulfoxide, sulfolane or acetonitrile and the like, at temperatures between 0 °C and boiling point of solvent, by procedures well known to those skilled in the art. Such reactions are known in literature by name of S_{N}Ar reaction.

Alternatively compounds of formula **XVIII** may be prepared by Chan-Lam coupling, which involves for example, reacting compounds of formula **XIX,** with compounds of formula **XXI,** wherein Yb₁ can be a boron-derived functional group, such as for example B(OH)₂ or B(ORb₁)₂ wherein Rb₁ can be a C₁-C₄alkyl group or the two groups ORb₁ can form together with the boron atom a five membered ring, as for example a pinacol boronic ester. The reaction may be catalyzed by a copper catalyst, for example Cu(OAc)₂, Cul, CuBr₂, CuCl amongst other copper based catalyst in presence of a base, like pyridine, sodium carbonate, tripotassium phosphate or cesium fluoride, in a solvent or a solvent mixture, like, for example dioxane, dichloromethane, acetonitrile, N,N-dimethyl-formamide, a mixture of 1,2-dimethoxyethane and water or of dioxane/water, or of toluene/water, under inert atmosphere or under oxygen atmosphere or under air. The reaction temperature can preferentially range from room temperature to the boiling point of the reaction mixture, or the reaction may be performed under microwave irradiation. Such Chan-Lam coupling reactions are well known to those skilled in the art.

The following non-limiting examples provide specific synthesis methods for representative compounds of the present invention, as referred to in Table 1 below.

### Example 1 Synthesis of 5-chloro-2-[[5-(trifluoromethyl)-2-[4-(trifluoromethyl)phenyl]pyrazol-3-yl]methyl]pyrimidine (Compound 1.001)

### Step 1: Synthesis of ethyl 6,6,6-trifluoro-3,5-dioxo-hexanoate

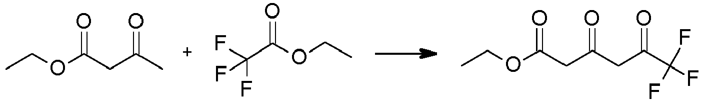

A solution of diisopropylamine (3.2 mL, 23 mmol) in tetrahydrofuran (19 mL) was placed under an atmosphere of nitrogen, cooled over ice and n-butyllithium (2.5 M in hexanes) (9.2 mL, 23 mmol) was added dropwise. The resulting pale-yellow mixture was allowed to stir for 30 minutes at 0°C.

To the above mixture was added dropwise ethyl acetoacetate (1.00 mL, 7.91 mmol). The reaction mixture was stirred at 0°C for one hour before being cooled to -78°C upon which ethyl trifluoroacetate (1.20 mL, 10.1 mmol) was added dropwise. The resulting reaction mixture was stirred for 3 hours, over which time it warmed to approximately 0°C.

The reaction was quenched with 1 M hydrochloric acid, diluted with water and extracted with dichloromethane and concentrated to give ethyl 6,6,6-trifluoro-3,5-dioxo-hexanoate which was used without further purification.

### Step 2: Synthesis of ethyl 2-[5-(trifluoromethyl)-2-[4-(trifluoromethyl) phenyl]pyrazol-3-yl]acetate

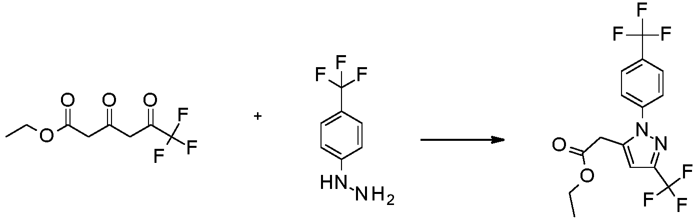

A solution of ethyl 6,6,6-trifluoro-3,5-dioxo-hexanoate in acetic acid (15 mL) was treated with 4- (trifluoromethyl)phenylhydrazine (1.35 g, 7.66 mmol) and stirred at room temperature for one hour then diluted with water and extracted with tert-butylmethyl ether and concentrated. The residues were subjected to flash column chromatography to give ethyl 2-[5-(trifluoromethyl)-2-[4-(trifluoromethyl)phenyl] pyrazol-3-yl]acetate as an orange solid (1.708 g, 53%).

¹H NMR (400 MHz, DMSO-d6) δ = 7.96 (d, 2H), 7.82 (d, 2H), 6.98 (s, 1H), 4.08 (s, 2H), 3.95 (q, 2H), 1.00 (t, 3H)

### Step 3: Synthesis of ethyl 2-(5-chloropyrimidin-2-yl)-2-[5-(trifluoromethyl)-2-[4-(trifluoromethyl)phenyl]pyrazol-3-yl]acetate

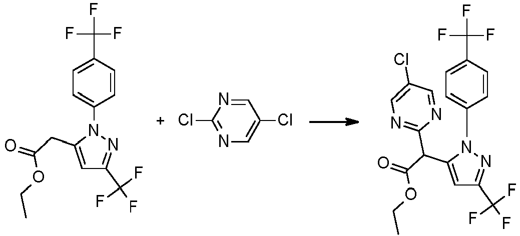

A mixture of ethyl 2-[5-(trifluoromethyl)-2-[4-(trifluoromethyl)phenyl]pyrazol-3-yl]acetate (109 mg, 0.2827 mmol), 2, 5-dichloropyrimidine (210 mg, 1.41 mmol) and sodium tert-butoxide (189 mg, 1.91 mmol) was placed under an atmosphere of nitrogen and diluted with sulfolane (1.4 mL). The mixture was warmed to 40°C and was stirred for 2 hours.

The reaction mixture was allowed to cool to room temperature and diluted with water and brine and extracted with tert-butylmethyl ether and concentrated. The residues were subjected to flash column chromatography to give ethyl 2-(5-chloropyrimidin-2-yl)-2-[5-(trifluoromethyl)-2-[4-(trifluoromethyl)phenyl]pyrazol-3-yl]acetate as a yellow oil (110 mg, 65%)

¹H NMR (400 MHz, CDCl₃) δ = 8.69 (s, 2H), 7.83 - 7.76 (m, 2H), 7.71 - 7.66 (m, 2H), 6.86 (s, 1H), 5.40 (s, 1H), 4.26 - 4.17 (m, 2H), 1.21 (t, 3H)

### Step 4: Synthesis of 5-chloro-2-[[5-(trifluoromethyl)-2-[4-(trifluoromethyl) phenyl]pyrazol-3-yl]methyl]pyrimidine

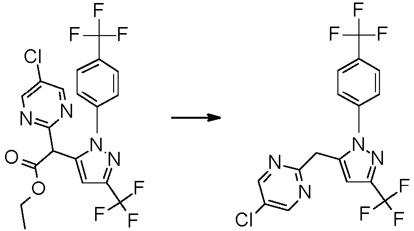

A solution of ethyl 2-(5-chloropyrimidin-2-yl)-2-[5-(trifluoromethyl)-2-[4-(trifluoromethyl)phenyl]pyrazol-3-yl]acetate (110 mg, 0.184 mmol) in ethanol (0.6 mL) was treated with hydrochloric acid (6 mol/L) in deionized water (1.0 mL) and the resulting mixture was irradiated under microwave radiation to 110°C for 45 minutes.

The mixture was diluted with water, extracted with ethyl acetate and concentrated. The residues were subjected to column chromatography to give 5-chloro-2-[[5-(trifluoromethyl)-2-[4-(trifluoromethyl)phenyl]pyrazol-3-yl]methyl]pyrimidine as a beige solid (51 mg, 65%).

¹H NMR (400 MHz, CDCl₃) δ = 8.64 (s, 2H), 7.80 - 7.70 (m, 4H), 6.61 (s, 1H), 4.38 (s, 2H)

### Example 2: Preparation of 5-bromo-2-[[5-(trifluoromethyl)-2-[4-(trifluoromethyl)phenyl]pyrazol-3-yl]methyl]pyrimidine (1.002)

### Step 1: Preparation of ethyl 2-(5-bromopyrimidin-2-yl)-2-[5-(trifluoromethyl)-2-[4-(trifluoromethyl)phenyl]pyrazol-3-yl]acetate (11)

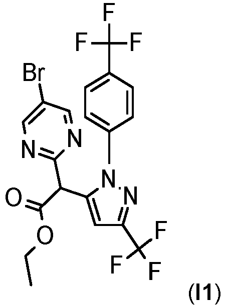

To a solution of ethyl 2-[5-(trifluoromethyl)-2-[4-(trifluoromethyl)phenyl]pyrazol-3-yl]acetate (352 mg, 0.913 mmol) as prepared in step 2 in the preparation of example 1 and 1,8-diazabicyclo[5.4.0]undec-7-ene (420 µL, 2.755 mmol) in sulfolane (2.0 mL) was added 5-bromo-2-chloropyrimidine (533 mg, 2.756 mmol) and the reaction mixture was stirred at room temperature for 2 hours. The mixture was treated with additional 1,8-diazabicyclo[5.4.0]undec-7-ene (140 µL, 0.918 mmol) and 5-bromo-2-chloropyrimidine (182 mg, 0.941 mmol) and was stirred for a further 2.5 hours. The mixture was diluted with water, acidified with hydrochloric acid and extracted with tert-butylmethyl ether. The combined organics were concentrated and subjected to column chromatography on silica gel using 0-20% ethyl acetate in cyclohexane to give ethyl 2-(5-bromopyrimidin-2-yl)-2-[5-(trifluoromethyl)-2-[4-(trifluoromethyl)phenyl] pyrazol-3-yl]acetate 11 (425 mg, 76%). ¹H NMR (400 MHz, CDCl₃) δ = 8.78 (s, 2H), 7.79 (d, 2H), 7.68 (d, 2H), 6.86 (s, 1H), 5.37 (s, 1H), 4.26 - 4.17 (m, 2H), 1.21 (t, 3H).

### Step 2: Preparation of 5-bromo-2-[[5-(trifluoromethyl)-2-[4-(trifluoromethyl)phenyl]pyrazol-3-yl]methyl]pyrimidine (1.002)

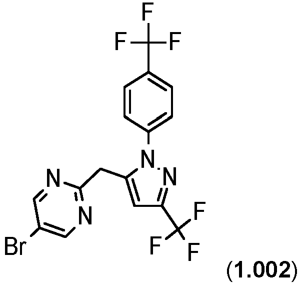

To a solution of ethyl 2-(5-bromopyrimidin-2-yl)-2-[5-(trifluoromethyl)-2-[4-(trifluoromethyl)phenyl]pyrazol-3-yl]acetate 11 (425 mg, 0.691 mmol) in methanol (3.5 mL) was added aqueous sodium hydroxide (2 M, 3.5 mL, 7.0 mmol) and the reaction mixture was stirred at 50°C for an hour. The mixture was cooled, diluted with water and extracted with ethyl acetate. The combined organics were concentrated and subjected to reverse-phase column chromatography on C-18 silica gel using 50-100% acetonitrile in water, both with 0.1% formic acid, to give 5-bromo-2-[[5-(trifluoromethyl)-2-[4-(trifluoromethyl)phenyl]pyrazol-3-yl]methyl]pyrimidine **1.002** (259 mg, 79%). ¹H NMR (400 MHz, CDCl₃) δ = 8.73 (s, 2H), 7.79 - 7.70 (m, 4H), 6.61 (s, 1H), 4.35 (s, 2H).

### Example 3: Preparation of 5-bromo-2-[[4-chloro-5-(trifluoromethyl)-2-[4-(trifluoromethyl)phenyl]pyrazol-3-yl]methyl]pyrimidine (1.034)

A solution of 5-bromo-2-[[5-(trifluoromethyl)-2-[4-(trifluoromethyl)phenyl]pyrazol-3-yl]methyl]pyrimidine 1.002 (154 mg, 0.307 mmol) and N-chlorosuccinimide (50 mg, 0.367 mmol) in acetonitrile (1.5 mL) was stirred at 80°C for 7 hours. The mixture was cooled, concentrated and subjected to column chromatography on silica gel using 0-40% ethyl acetate in cyclohexane to give 5-bromo-2-[[4-chloro-5-(trifluoromethyl)-2-[4-(trifluoromethyl)phenyl]pyrazol-3-yl]methyl]pyrimidine 1.034 (103 mg, 66%). ¹H NMR (400 MHz, CDCl₃) δ = 8.80 (s, 2H), 7.76 - 7.64 (m, 4H), 5.38 (s, 2H).

### Example 4: Preparation of 5-[(5-chloropyrimidin-2-yl)methyl]-1-(3,4-difluorophenyl)pyrazole-3-carbonitrile (1.025)

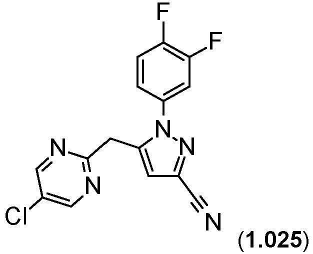

### Step 1: Preparation of 1-(3,4-difluorophenyl)pyrazole-3-carbonitrile (12)

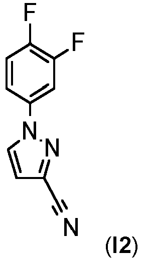

To a mixture of 1H-pyrazole-3-carbonitrile (519 mg, 5.58 mmol), 3,4-difluorophenylboronic acid (1.79 g, 10.8 mmol) and copper(II) acetate (1.60 g, 8.81 mmol) was added dichloromethane (11 mL) and pyridine (880 µL, 10.8 mmol) and the reaction mixture was stirred rapidly at room temperature under air for 20 hours. The mixture was concentrated and subjected to column chromatography on silica gel using 0-40% ethyl acetate in cyclohexane to give 1-(3,4-difluorophenyl)pyrazole-3-carbonitrile 12 (964 g, 80%). ¹H NMR (400 MHz, CDCl₃) δ = 7.93 (d, 1H), 7.65 - 7.58 (m, 1H), 7.47 - 7.41 (m, 1H), 7.36 - 7.27 (m, 1H), 6.88 (d, 1H).

### Step 2: Preparation of 1-(3,4-difluorophenyl)-5-formyl-pyrazole-3-carbonitrile (13)

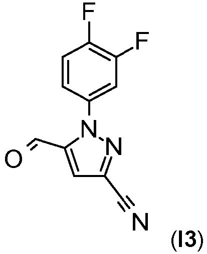

To a solution of 1-(3,4-difluorophenyl)pyrazole-3-carbonitrile **12** (1.238 g, 5.73 mmol) in tetrahydrofuran (14.0 mL) was added lithium diisopropylamide (2.0 M in THF/ heptane/ethylbenzene, 4.6 mL, 9.2 mmol) at -78°C and the resulting mixture was stirred for 45 minutes. To this mixture was then added N,N-dimethylformamide (0.9 mL, 10 mmol) and the mixture was stirred an hour. The mixture was quenched with 1 M hydrochloric acid, diluted with water and extracted with ethyl acetate. The combined organics were concentrated and subjected to column chromatography on silica gel using 0-40% ethyl acetate in cyclohexane to give 1-(3,4-difluorophenyl)-5-formyl-pyrazole-3-carbonitrile I**3** (638 mg, 45%). ¹H NMR (400 MHz, CDCl₃) δ = 9.88 (s, 1H), 7.46 (s, 1H), 7.45 - 7.34 (m, 2H), 7.30 - 7.27 (m, 1H).

### Step 3: Preparation of 5-[(5-chloropyrimidin-2-yl)-hydroxy-methyl]-1-(3,4-difluorophenyl)pyrazole-3-carbonitrile (14)

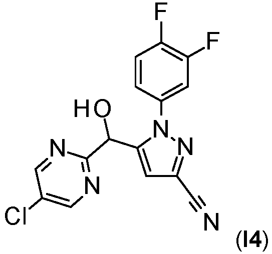

To a solution of 5-chloro-2-iodopyrimidine (387 mg, 1.61 mmol) in toluene (7 mL) was added n-butyllithium (2.5 M in hexanes, 0.55 mL, 1.4 mmol) at -78°C. The resulting mixture was stirred for 15 minutes before being treated with a solution of 1-(3,4-difluorophenyl)-5-formyl-pyrazole-3-carbonitrile 13 (285 mg, 1.16 mmol) in toluene (4 mL) and stirred for a further 45 minutes. The mixture was quenched with 0.5 M hydrochloric acid and extracted with ethyl acetate. The combined organics were concentrated and subjected to column chromatography on silica gel using 0-40% ethyl acetate in cyclohexane to give 5-[(5-chloropyrimidin-2-yl)-hydroxy-methyl]-1-(3,4-difluorophenyl)pyrazole-3-carbonitrile I**4** (232 mg, 52%). ¹H NMR (400 MHz, CDCl₃) δ = 8.74 (s, 2H), 7.68 (ddd, 1H), 7.56 - 7.50 (m, 1H), 7.36 - 7.29 (m, 1H), 6.46 (s, 1H), 5.85 (d, 1H), 4.62 (d, 1H).

### Step 4: Preparation of 5-[(5-chloropyrimidin-2-yl)methyl]-1-(3,4-difluorophenyl)pyrazole-3-carbonitrile (1.025)

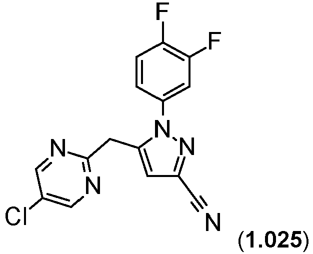

To a solution of 5-[(5-chloropyrimidin-2-yl)-hydroxy-methyl]-1-(3,4-difluorophenyl)pyrazole-3-carbonitrile I**4** (232 mg, 0.60 mmol), imidazole (70 mg, 1.03 mmol) and triphenylphosphine (404 mg, 1.51 mmol) in tetrahydrofuran (4.6 mL) was added dropwise a solution of iodine (208 mg, 0.82 mmol) in tetrahydrofuran (1.2 mL). The mixture was stirred for five minutes before being quenched with aqueous sodium thiosulphate, diluted with water and extracted with ethyl acetate. The combined organics were concentrated and subjected to column chromatography on silica gel using 0-40% ethyl acetate in cyclohexane. The product-rich fractions were concentrated and subjected to reverse-phase column chromatography on C-18 silica gel using 50-100% acetonitrile in water, both with 0.1% formic acid, to give 5-[(5-chloropyrimidin-2-yl)methyl]-1-(3,4-difluorophenyl)pyrazole-3-carbonitrile 1.025 (148 mg, 71%). ¹H NMR (400 MHz, CDCl₃) δ = 8.64 (s, 2H), 7.53 - 7.45 (m, 1H), 7.36 - 7.27 (m, 2H), 6.72 (s, 1H), 4.34 (s, 2H).

### Example 5: Preparation of 4-[5-[(5-chloropyrimidin-2-yl)methyl]-3-(trifluoromethyl)pyrazol-1-yl]benzonitrile (1.007)

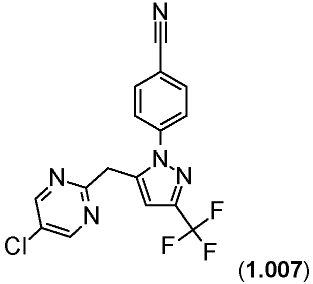

### Step 1: Preparation of ethyl 6,6,6-trifluoro-3,5-dioxo-hexanoate (I5)

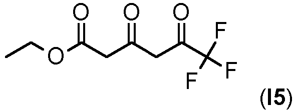

To an ice-cooled solution of diisopropylamine (22 mL, 157 mmol) in THF (110 mL) in a 250 mL round bottomed flask was added n-butyllithium (2.5 M in hexanes, 65 mL, 160 mmol) over approximately 30 minutes. The mixture was stirred for 30 minutes, treated dropwise with ethyl acetoacetate (6.8 mL, 54 mmol) and was stirred for a further 45 minutes at 0°C. The reaction mixture was then cooled to -78°C, treated dropwise with ethyl trifluoroacetate (8.3 mL, 70 mmol) and stirred for 3 hours. The mixture was quenched with hydrochloric acid, diluted with water and extracted with ethyl acetate. The combined organics were dried and concentrated to give **I5** as a resultant oil which was used as such in the next step.

### Step 2: Preparation of ethyl 2-[2-[(4-methoxyphenyl)methyl]-5-(trifluoromethyl)pyrazol-3-yl]acetate (I6)

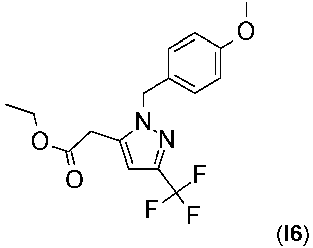

To a solution of **I5** prepared in step 1 in acetic acid (90 mL) in a 100 mL round bottomed flask was added (4-methoxybenzyl)hydrazine hydrochloride (10.56 g, 54.29 mmol) and the resulting mixture was allowed to stir for 1.5 hours at room temperature. The mixture was diluted with tert-butylmethyl ether and washed with water and brine. The organics were concentrated and subjected to column chromatography on silica gel using 0-30% ethyl acetate in cyclohexane to give ethyl 2-[2-[(4-methoxyphenyl)methyl]-5-(trifluoromethyl)pyrazol-3-yl]acetate **I6** (11.97 g, 59% yield). ¹H NMR (400 MHz, CDCl₃) δ = 7.10 - 7.04 (m, 2H), 6.88 - 6.83 (m, 2H), 6.50 (s, 1H), 5.35 (s, 2H), 4.12 (q, 2H), 3.79 (s, 3H), 3.54 (s, 2H), 1.24 (t, 3H).

### Step 3: Preparation of ethyl 2-(5-chloropyrimidin-2-yl)-2-[2-[(4-methoxyphenyl)methyl]-5-(trifluoromethyl)pyrazol-3-yl]acetate (17)

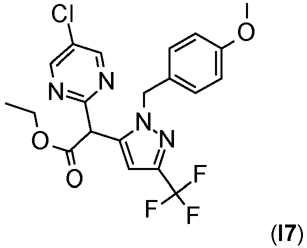

To a solution of ethyl 2-[2-[(4-methoxyphenyl)methyl]-5-(trifluoromethyl)pyrazol-3-yl]acetate **16** (4.22 g, 11.71 mmol) in DMSO (40 mL) in a 250 mL round bottomed flask was added 2,5-dichloropyrimidine (3.71 g, 24.9 mmol) and potassium phosphate tribasic (7.86 g, 36.3 mmol) and the reaction mixture was stirred at 60°C for 3.5 hours. The mixture was allowed to stand at room temperature overnight and then was stirred at 60°C for an additional hour. The mixture was cooled to room temperature, diluted with water and extracted with tert-butylmethyl ether. The combined organics were concentrated and subjected to column chromatography on silica gel using 0-40% ethyl acetate in cyclohexane to give ethyl 2-(5-chloropyrimidin-2-yl)-2-[2-[(4-methoxyphenyl)methyl]-5-(trifluoromethyl)pyrazol-3-yl]acetate **17** (5.19 g, 73% yield). ¹H NMR (400 MHz, CDCl₃) δ = 8.55 (s, 2H), 6.98 - 6.92 (m, 2H), 6.79 - 6.74 (m, 2H), 6.69 (s, 1H), 5.45 - 5.31 (m, 3H), 4.20 - 4.11 (m, 2H), 3.77 (s, 3H), 1.21 - 1.16 (m, 3H).

### Step 4: Preparation of 5-chloro-2-[[2-[(4-methoxyphenyl)methyl]-5-(trifluoromethyl)pyrazol-3-yl]methyl]pyrimidine (18)

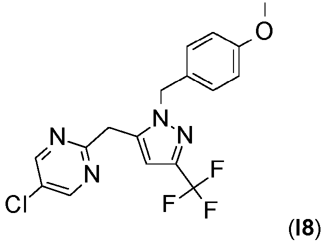

To a solution of ethyl 2-(5-chloropyrimidin-2-yl)-2-[2-[(4-methoxyphenyl)methyl]-5-(trifluoromethyl)pyrazol-3-yl]acetate 17 (5.19 g, 8.56 mmol) in methanol (40 mL) in a 250 mL round bottomed flask was added 2 M sodium hydroxide (40 mL) and the reaction mixture was stirred at 60°C for 1.5 hours. The mixture was cooled to room temperature and concentrated to half volume. The residues were then acidified with hydrochloric acid and extracted with ethyl acetate. The combined organics were dried and concentrated to give 5-chloro-2-[[2-[(4-methoxyphenyl)methyl]-5-(trifluoromethyl)pyrazol-3-yl]methyl]pyrimidine **I8** (3.92 g, quantitative yield). ¹H NMR (400 MHz, CDCl₃) δ = 8.55 (s, 2H), 7.05 - 6.95 (m, 2H), 6.84 - 6.75 (m, 2H), 6.47 (s, 1H), 5.40 (s, 2H), 4.22 (s, 2H), 3.78 (s, 3H).

### Step 5: Preparation of 5-chloro-2-[[3-(trifluoromethyl)-1H-pyrazol-5-yl]methyl]pyrimidine (19)

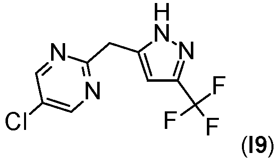

A solution of 5-chloro-2-[[2-[(4-methoxyphenyl)methyl]-5-(trifluoromethyl)pyrazol-3-yl]methyl]pyrimidine **I8** (3.92 g, 8.56 mmol) in 2,2,2-trifluoroacetic acid (14 mL) in a 100 mL round bottomed flask was stirred at 90°C for one hour. The mixture was cooled to room temperature, slowly transferred into an aqueous sodium bicarbonate solution and then extracted with ethyl acetate. The combined organics were concentrated and subjected to column chromatography on silica gel using 0-60% ethyl acetate in cyclohexane to give 5-chloro-2-[[3-(trifluoromethyl)-1H-pyrazol-5-yl]methyl]pyrimidine 19 (2.365 g, 98% yield). ¹H NMR (400 MHz, CDCl₃) δ = 8.70 (s, 2H), 6.49 (s, 1H), 4.41 (s, 2H).

### Step 6: Preparation of 4-[5-[(5-chloropyrimidin-2-yl)methyl]-3-(trifluoromethyl) pyrazol-1-yl]benzonitrile (1.007)

To a mixture of 5-chloro-2-[[3-(trifluoromethyl)-1H-pyrazol-5-yl]methyl]pyrimidine **I9** (101 mg, 0.346 mmol), (4-cyanophenyl)boronic acid (126 mg, 0.857 mmol) and copper(II) acetate (101 mg, 0.556 mmol) was added molecular sieves 4Å, dichloromethane (2.3 mL) and pyridine (55 µL, 0.676 mmol) and the reaction mixture was stirred rapidly at room temperature under air for 24 hours. The combined organics were concentrated and subjected to column chromatography on silica gel using 0-40% ethyl acetate in cyclohexane. The product-rich fractions were concentrated and subjected to reverse-phase column chromatography on C-18 silica gel using 40-100% acetonitrile in water, both with 0.1% formic acid, to give 5-[(5-chloropyrimidin-2-yl)methyl]-1-(3,4-difluorophenyl)pyrazole-3-carbonitrile **1.007** (11 mg, 8%). ¹H NMR (400 MHz, CDCl₃) δ = 8.64 (s, 2H), 7.83 - 7.75 (m, 4H), 6.63 (s, 1H), 4.39 (s, 2H).

### Example 6: Preparation of 5-chloro-2-[[2-(p-tolyl)-5-(trifluoromethyl)pyrazol-3-yl]methyl]pyrimidine (1.037)

A suspension of 5-chloro-2-[[3-(trifluoromethyl)-1H-pyrazol-5-yl]methyl]pyrimidine **I9** (46 mg, 0.158 mmol), 4-iodotoluene (76 mg, 0.349 mmol), copper(I) iodide (4 mg, 0.021 mmol), trans-N,N'-dimethyl-1,2-cyclohexanediamine bis-(methanesulfonic acid) (25 mg, 0.075 mmol) and potassium carbonate (71 mg, 0.514 mmol) in 1,4-dioxane (0.6 mL) was irradiated under microwave irradiation to 150°C for 5 hours. The mixture was diluted with water and extracted with ethyl acetate. The combined organics were concentrated and subjected to column chromatography on silica gel using 0-40% ethyl acetate in cyclohexane. The product-rich fractions were concentrated and subjected to reverse-phase column chromatography on C-18 silica gel using 40-100% acetonitrile in water, both with 0.1% formic acid, to give 5-chloro-2-[[2-(p-tolyl)-5-(trifluoromethyl)pyrazol-3-yl]methyl]pyrimidine **1.037** (9 mg, 15%). ¹H NMR (400 MHz, CDCl₃) δ = 8.62 (s, 2H), 7.39 - 7.34 (m, 2H), 7.28 - 7.23 (m, 2H), 6.53 (s, 1H), 4.32 (s, 2H), 2.41 (s, 3H).

### Example 7: Preparation of 5-chloro-2-[[2-(6-chloro-5-fluoro-3-pyridyl)-5-(trifluoromethyl)pyrazol-3-yl]methyl]pyrimidine 1.026.

### Step 1: Preparation of tert-butyl N-(6-chloro-5-fluoro-3-pyridyl)carbamate 110-a.

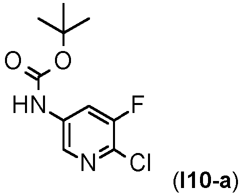

To a mixture of 5-bromo-2-chloro-3-fluoropyridine (1.06 g, 5.04 mmol), tert-butyl carbamate (768 mg, 6.56 mmol), palladium(II) acetate (40 mg, 0.173 mmol), 4,5-bis(diphenylphosphino)-9,9-dimethylxanthene (182 mg, 0.305 mmol) and potassium carbonate (1.33 g, 9.62 mmol) was added 1,4-dioxane (12 mL) and the mixture was stirred at 110°C for 7 hours. The mixture was cooled, diluted with water and extracted with ethyl acetate. The combined organics were concentrated and subjected to column chromatography on silica gel using 0-40% ethyl acetate in cyclohexane to give tert-butyl N-(6-chloro-5-fluoro-3-pyridyl)carbamate 110-a (1.062 g, 77% yield). ¹H NMR (400 MHz, CDCl₃) δ = 8.06 (br d, 1H), 7.98 (d, 1H), 6.64 (br s, 1H), 1.53 (s, 9H).

### Step 2: Preparation of 6-chloro-5-fluoro-pyridin-3-amine trifluoroacetate 110-b.

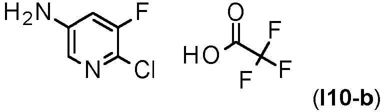

To a solution of tert-butyl N-(6-chloro-5-fluoro-3-pyridyl)carbamate **I10-a** (1.062 g, 3.875 mmol) in dichloromethane (8 mL) was added trifluoroacetic acid (3.0 mL, 39 mmol) and the resulting mixture was stirred at room temperature for an hour. On completion the mixture was concentrated to concentrated to give 6-chloro-5-fluoro-pyridin-3-amine trifluoroacetate **I10-b.** as a resultant gum which was used as such in the next step.

### Step 3: Preparation of (6-chloro-5-fluoro-3-pyridyl)hydrazine;hydrochloride 110-c.

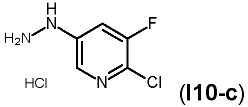

To a solution of **I10-b** prepared in step 2 in aqueous hydrochloric acid (6.5 mL, 6 M) was added a solution of sodium nitrite (284 mg, 4.116 mmol) in water (2 mL) at 0°C. The resulting mixture was stirred for 15 minutes and treated with a solution of tin chloride (1.96 g, 10.3 mmol) in aqueous hydrochloric acid (6.5 mL, 6 M). The resulting mixture was stirred at 0 °C for 15 minutes. On completion, the mixture was basified with aqueous sodium hydroxide and extracted with ethyl acetate. The combined organics were dried and concentrated. The residues were re-dissolved in tert-butylmethyl ether and treated with hydrochloric acid (1.25 M in EtOH, 3.5 mL, 4.4 mmol). The mixture was stirred for an hour and the precipitate was collected by filtration to give (6-chloro-5-fluoro-3-pyridyl)hydrazine hydrochloride 110-c (558 mg, 69% yield). ¹H NMR (400 MHz, DMSO-d₆) δ = 10.83 (br s, 3H), 8.96 (br s, 1H), 7.99 - 7.94 (m, 1H), 7.58 - 7.47 (m, 1H).

### Step 4: Preparation of ethyl 2-[2-(6-chloro-5-fluoro-3-pyridyl)-5-(trifluoromethyl)pyrazol-3-yl]acetate 111.

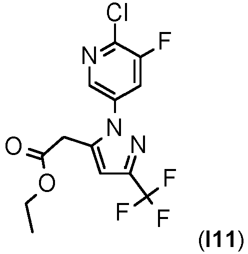

To a solution of (6-chloro-5-fluoro-3-pyridyl)hydrazine hydrochloride 110-c (242 mg, 1.161 mmol) in aqueous hydrochloric acid (6 mL, 6 M) was added a solution of ethyl 6,6,6-trifluoro-3,5-dioxo-hexanoate **15** (688 mg, 1.947 mmol) in ethanol (6 mL) and the mixture was stirred at room temperature for an hour. The mixture was diluted with water and extracted with ethyl acetate. The combined organics were concentrated and subjected to column chromatography on silica gel using 0-30% ethyl acetate in cyclohexane to give ethyl 2-[2-(6-chloro-5-fluoro-3-pyridyl)-5-(trifluoromethyl)pyrazol-3-yl]acetate **I11** (233 mg, 34% yield, 60% purity). ¹H NMR (400 MHz, chloroform) δ = 8.42 (d, 1H), 7.80 (dd, 1H), 6.72 (s, 1H), 4.18 (q, 2H), 3.75 (s, 2H), 1.26 (t, 3H).

### Step 5: Preparation of ethyl 2-[2-(6-chloro-5-fluoro-3-pyridyl)-5-(trifluoromethyl)pyrazol-3-yl]-2-(5-chloropyrimidin-2-yl)acetate 112.

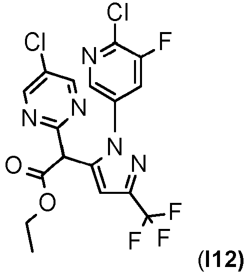

To a solution of ethyl 2-[2-(6-chloro-5-fluoro-3-pyridyl)-5-(trifluoromethyl)pyrazol-3-yl]acetate **I11** (370 mg, 0.631 mmol) and 2,5-dichloropyrimidine (366 mg, 2.457 mmol) in dimethylsulfoxide (2.0 mL) was added potassium phosphate tribasic (693 mg, 3.20 mmol) and the mixture was stirred at 80 °C for an hour. The mixture was cooled, diluted with 0.5 M hydrochloric acid and extracted with tert-butylmethyl ether. The combined organics were concentrated and subjected to column chromatography on silica gel using 0-25% ethyl acetate in cyclohexane to give ethyl 2-[2-(6-chloro-5-fluoro-3-pyridyl)-5-(trifluoromethyl)pyrazol-3-yl]-2-(5-chloropyrimidin-2-yl)acetate **I12** (174 mg, 47% yield). ¹H NMR (400 MHz, chloroform) δ = 8.70 (s, 2H), 8.50 (d, 1H), 7.86 (dd, 1H), 6.91 (s, 1H), 5.35 (s, 1H), 4.30 - 4.17 (m, 2H), 1.23 (t, 3H).

### Step 6: Preparation of 5-chloro-2-[[2-(6-chloro-5-fluoro-3-pyridyl)-5-(trifluoromethyl)pyrazol-3-yl]methyl]pyrimidine 1.026.

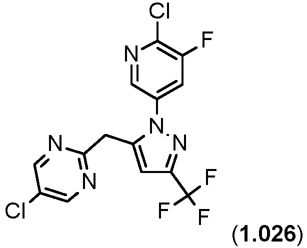

To a solution of ethyl 2-[2-(6-chloro-5-fluoro-3-pyridyl)-5-(trifluoromethyl)pyrazol-3-yl]-2-(5-chloropyrimidin-2-yl)acetate I**12** (309 mg, 0.585 mmol) in tetrahydrofuran (2.5 mL) was added aqueous hydrochloric acid (6 M, 2.5 mL, 15 mmol) and the mixture was heated to 150°C for 40 minutes under microwave irradiation. The reaction mixture was diluted with water and extracted with ethyl acetate. The combined organics were concentrated and subjected to reverse-phase column chromatography on C-18 silica gel using 50-100% acetonitrile in water, both with 0.1% formic acid. The product-rich fractions were concentrated and subjected column chromatography on silica gel using 0-40% ethyl acetate in cyclohexane to give 5-chloro-2-[[2-(6-chloro-5-fluoro-3-pyridyl)-5-(trifluoromethyl)pyrazol-3-yl]methyl]pyrimidine **1.026** (180 mg, 74.61% Yield). ¹H NMR (400 MHz, chloroform) δ = 8.65 (s, 2H), 8.60 (d, 1H), 7.95 (dd, 1H), 6.65 (s, 1H), 4.38 (s, 2H).

### Example 8: Preparation of 5-chloro-2-[[5-(difluoromethyl)-2-(3,4-difluorophenyl)pyrazol-3-yl]methyl]pyrimidine 1.032

### Step 1: Preparation of ethyl 6,6-difluoro-3,5-dioxo-hexanoate 113-a.

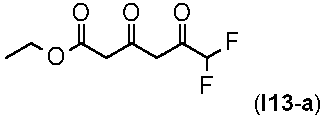

To an ice-cooled solution of lithium diisopropylamide (2.0 M in THF/ heptane/ethylbenzene, 45 mL, 90 mmol) THF (50 mL) was added dropwise ethyl acetoacetate (3.9 mL, 31 mmol) and the mixture was stirred for 30 minutes at 0°C. The reaction mixture was then cooled to -78°C, treated dropwise with ethyl difluoroacetate (4.2 mL, 40 mmol) and stirred for 2 hours. The mixture was quenched with hydrochloric acid, diluted with water and extracted with ethyl acetate. The combined organics were dried and concentrated to give 113-a as a resultant oil which was used as such in the next step assuming quantitative conversion.

### Step 2: Preparation of ethyl 2-[5-(difluoromethyl)-2-(3,4-difluorophenyl)pyrazol-3-yl]acetate I13-b.

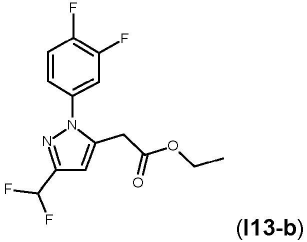

A solution of ethyl 6,6-difluoro-3,5-dioxo-hexanoate **I13-a** (1.6 g, 7.75 mmol) in acetic acid (12.5 mL) in a 50 mL round bottomed flask was treated with 3,4-difluorophenylhydrazine hydrochloride (1.44 g, 7.97 mmol) and was allowed to stir for 20 hours at room temperature. The reaction mixture was concentrated and subjected to column chromatography (0-40% ethyl acetate in cyclohexane). The fractions containing the product were combined and concentrated *in vacuo* to give ethyl 2-[5-(difluoromethyl)-2-(3,4-difluorophenyl)pyrazol-3-yl]acetate I**13-b** (1.299 g, 70% purity, 37.1% Yield). ¹H NMR (400 MHz, chloroform) δ = 7.44 - 7.20 (m, 3H), 6.70 (t, 1H), 6.63 (s, 1H), 4.16 (q, 2H), 3.70 (s, 2H), 1.25 (t, 3H).

### Step 3: Preparation of ethyl 2-(5-chloropyrimidin-2-yl)-2-[5-(difluoromethyl)-2-(3,4-difluorophenyl)pyrazol-3-yl]acetate 114.

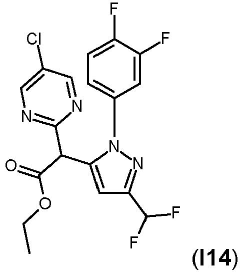

To a solution of ethyl 2-[5-(difluoromethyl)-2-(3,4-difluorophenyl)pyrazol-3-yl]acetate **I13-b** (318 mg, 0.704 mmol) and 2,5-dichloropyrimidine (251 mg, 1.685 mmol) in dimethylsulfoxide (2.0 mL) was added potassium phosphate tribasic (572 mg, 2.641 mmol) and the mixture was stirred at 80°C for 1.5 hours. The mixture was cooled acidified with hydrochloric acid, diluted with water and extracted with tert-butylmethyl ether. The combined organics were concentrated and subjected to column chromatography on silica gel using 0-30% ethyl acetate in cyclohexane to give ethyl 2-(5-chloropyrimidin-2-yl)-2-[5-(difluoromethyl)-2-(3,4-difluorophenyl)pyrazol-3-yl]acetate **I14** (260 mg, 60% Yield). ¹H NMR (400 MHz, chloroform) δ = 8.68 (s, 2H), 7.45 - 7.26 (m, 3H), 6.76 (s, 1H), 6.70 (t, 1H), 5.35 (s, 1H), 4.26 - 4.18 (m, 2H), 1.22 (t, 3H).

### Step 4: Preparation of 5-chloro-2-[[5-(difluoromethyl)-2-(3,4-difluorophenyl)pyrazol-3-yl]methyl]pyrimidine 1.032.

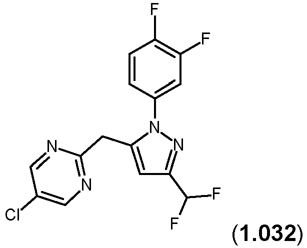

To a solution of ethyl 2-(5-chloropyrimidin-2-yl)-2-[5-(difluoromethyl)-2-(3,4-difluorophenyl)pyrazol-3-yl]acetate I**14** (304 mg, 0.496 mmol) in methanol (3 mL) was added aqueous sodium hydroxide (2 M, 3 mL) and the mixture was stirred at room temperature for 17 hours. The mixture was acidified with hydrochloric acid, diluted with water and extracted with ethyl acetate. The combined organics were concentrated and subjected to reverse-phase column chromatography on C-18 silica gel using 50-100% acetonitrile in water, both with 0.1% formic acid, to give 5-chloro-2-[[5-(difluoromethyl)-2-(4-fluorophenyl)pyrazol-3-yl]methyl]pyrimidine **1.032** (135 mg, 72% yield). ¹H NMR (400 MHz, chloroform) δ = 8.64 (s, 2H), 7.53 - 7.46 (m, 1H), 7.36 - 7.22 (m, 2H), 6.69 (t, 1H), 6.53 (s, 1H), 4.34 (s, 2H).

**Table 1 - Examples of herbicidal compounds of the present invention.**

| **COMPOUND** | **STRUCTURE** | **1H NMR** (400MHz, CDCI₃ unless otherwise stated) |
|---|---|---|
| 1.001 | 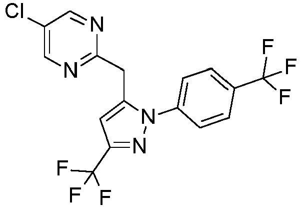 | 8.64 (s, 2H), 7.80 - 7.70 (m, 4H), 6.61 (s, 1H), 4.38 (s, 2H) |
| 1.002 | 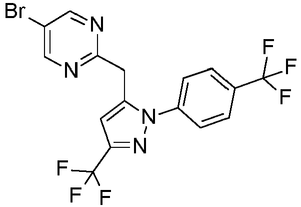 | 8.73 (s, 2H), 7.79 - 7.70 (m, 4H), 6.61 (s, 1H), 4.35 (s, 2H) |
| 1.003 | 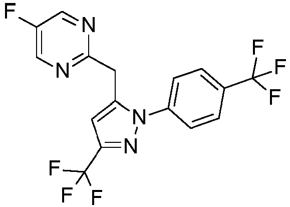 | 8.54 (s, 2H), 7.80 - 7.70 (m, 4H), 6.61 (s, 1H), 4.40 (s, 2H) |
| 1.004 | 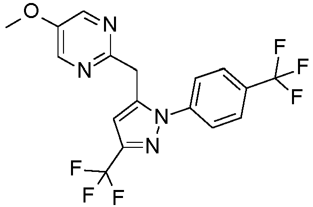 | 8.33 (s, 2H), 7.77- 7.74 (m, 4H), 6.58 (s, 1H), 4.34 (s, 2H), 3.91 (s, 3H) |
| 1.005 | 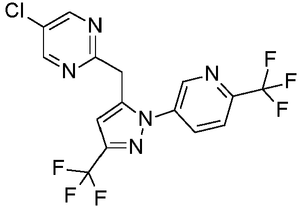 | |
| 1.006 | 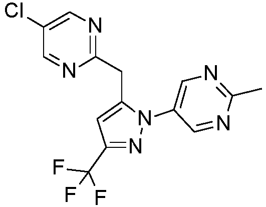 | |
| 1.007 | 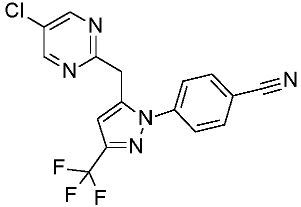 | 8.64 (s, 2H), 7.83 - 7.75 (m, 4H), 6.63 (s, 1H), 4.39 (s, 2H) |
| 1.008 | 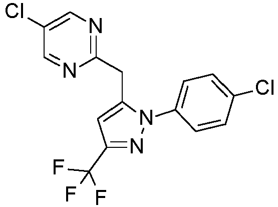 | 8.63 (s, 2H), 7.54 - 7.42 (m, 4H), 6.57 (s, 1H), 4.33 (s, 2H) |
| 1.009 | 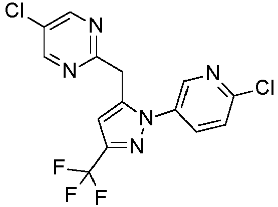 | 8.69 (dd, 1H), 8.64 (s, 2H), 7.96 (dd, 1H), 7.49 (dd, 1H), 6.64 (s, 1H), 4.35 (s, 2H) |
| 1.010 | 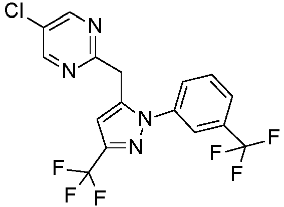 | |
| 1.011 | 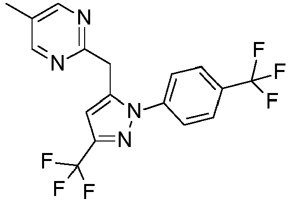 | 8.50 (s, 2H), 7.80-7.69 (m, 4H), 6.58 (s, 1H), 4.35 (s, 2H), 2.29 (s, 3H) |
| 1.012 | 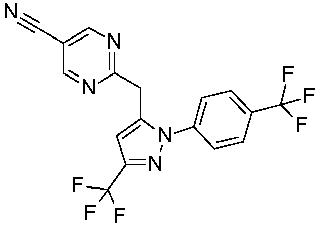 | 8.94 (s, 2H), 7.80 (d, 2H), 7.71 (d, 2H), 6.65 (s, 1H), 4.50 (s, 2H) |
| 1.013 | 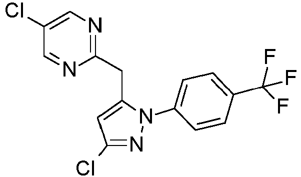 | |
| 1.014 | 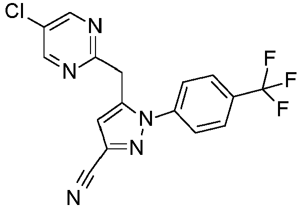 | |
| 1.015 | 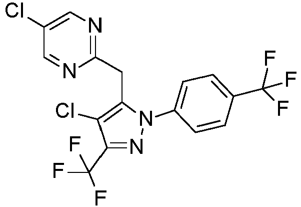 | |
| 1.016 | 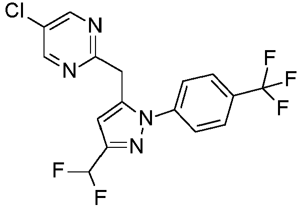 | 8.63 (s, 2H), 7.79 - 7.69 (m, 4H), 6.87 - 6.54 (m, 2H), 4.38 (s, 2H) |
| 1.017 | 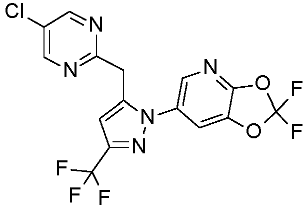 | |
| 1.018 | 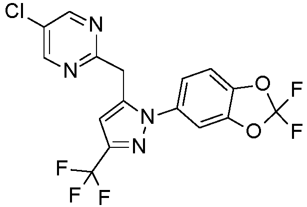 | |
| 1.019 | 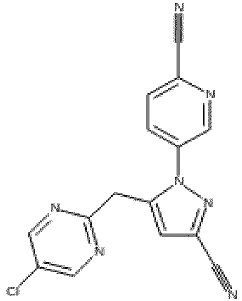 | 9.03 - 9.12 (m, 1 H) 8.65 (s, 2 H) 8.17 - 8.31 (m, 1 H) 7.89 (dd, 1 H) 6.83 (s, 1 H) 4.42 (s, 2 H) |
| 1.020 | 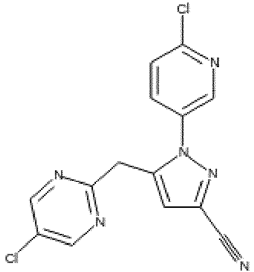 | 8.65 - 8.70 (m, 1 H) 8.60 - 8.64 (m, 2 H) 7.88 - 7.99 (m, 1 H) 7.46 - 7.54 (m, 1 H) 6.74 - 6.82 (m, 1 H) 4.31 - 4.41 (m, 2 H) |
| 1.021 | 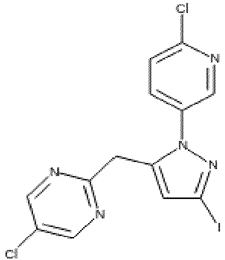 | 8.65 (d, 1 H) 8.63 (s, 2 H) 7.92 (dd, 1 H) 7.45 (d, 1 H) 6.52 (s, 1 H) 4.32 (s, 2 H) |
| 1.022 | 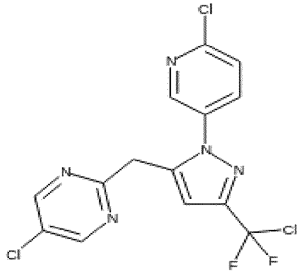 | 8.70 (d, 1H), 8.64 (s, 2H), 7.96 (dd, 1H), 7.48 (dd, 1H), 6.62 (s, 1H), 4.34 (s, 2H) |
| 1.023 | 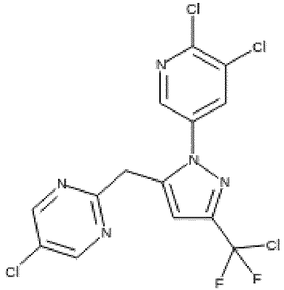 | 8.67 (d, 1H), 8.65 (d, 2H), 8.19 (d, 1H), 6.64 (s, 1H), 4.36 (s, 2H) |
| 1.024 | 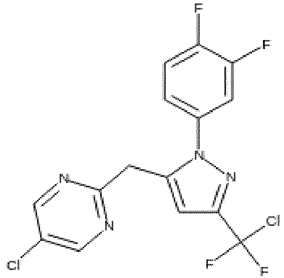 | 8.65 (s, 2H), 7.52 (ddd, 1H), 7.39 - 7.33 (m, 1H), 7.32 - 7.23 (m, 1H), 6.56 (s, 1H), 4.33 (s, 2H) |
| 1.025 | 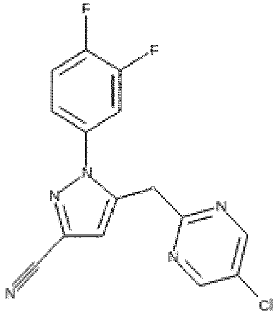 | 8.64 (s, 2H), 7.53 - 7.45 (m, 1H), 7.36 - 7.27 (m, 2H), 6.72 (s, 1H), 4.34 (s, 2H) |
| 1.026 | 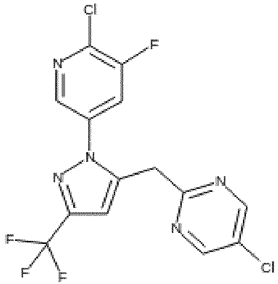 | 8.65 (s, 2H), 8.60 (d, 1H), 7.95 (dd, 1H), 6.65 (s, 1H), 4.38 (s, 2H) |
| 1.027 | 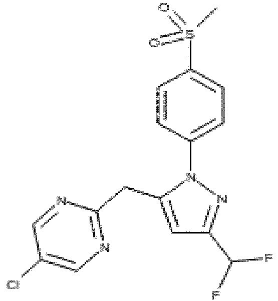 | 8.64 (s, 2H), 8.10 - 8.04 (m, 2H), 7.88 - 7.82 (m, 2H), 6.71 (t, 1H), 6.59 (s, 1H), 4.41 (s, 2H), 3.10 (s, 3H) |
| 1.028 | 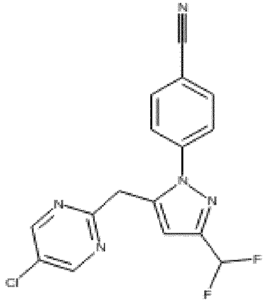 | 8.64 (s, 2H), 7.82 - 7.73 (m, 4H), 6.70 (t, 1H), 6.58 (s, 1H), 4.40 (s, 2H) |
| 1.029 | 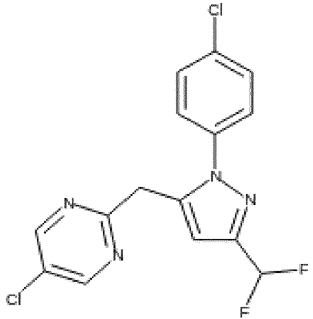 | 8.62 (s, 2H), 7.50 - 7.42 (m, 4H), 6.70 (t, 1H), 6.52 (s, 1H), 4.33 (s, 2H) |
| 1.030 | 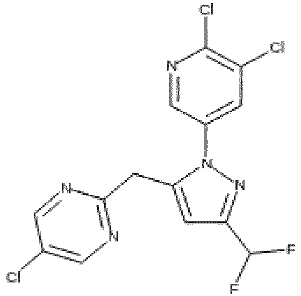 | 8.66 - 8.64 (m, 3H), 8.18 (d, 1H), 6.69 (t, 1H), 6.61 (s, 1H), 4.37 (s, 2H) |
| 1.031 | 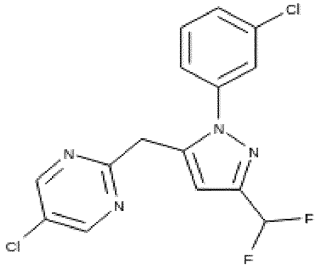 | 8.63 (s, 2H), 7.58 - 7.55 (m, 1H), 7.49 - 7.36 (m, 3H), 6.70 (t, 1H), 6.53 (s, 1H), 4.36 (s, 2H) |
| 1.032 | 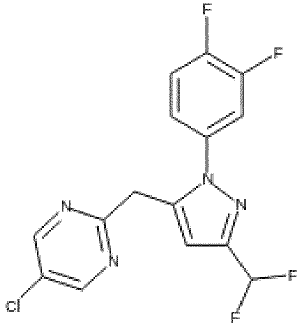 | 8.64 (s, 2H), 7.53 - 7.46 (m, 1H), 7.36 - 7.22 (m, 2H), 6.69 (t, 1H), 6.53 (s, 1H), 4.34 (s, 2H) |
| 1.033 | 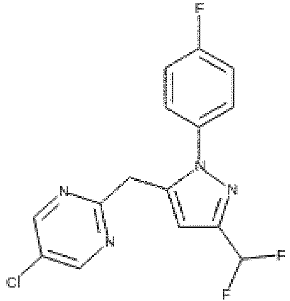 | 8.62 (s, 2H), 7.54 - 7.46 (m, 2H), 7.20 - 7.12 (m, 2H), 6.70 (t, 1H), 6.52 (s, 1H), 4.32 (s, 2H) |
| 1.034 | 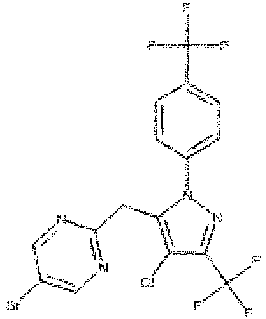 | 8.80 (s, 2H), 7.76 - 7.64 (m, 4H), 5.38 (s, 2H) |
| 1.035 | 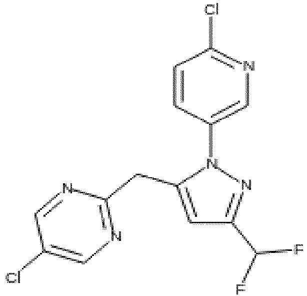 | 8.68 (dd, 1H), 8.63 (s, 2H), 7.94 (dd, 1H), 7.47 (dd, 1H), 6.70 (t, 1H), 6.59 (s, 1H), 4.35 (s, 2H) |
| 1.036 | 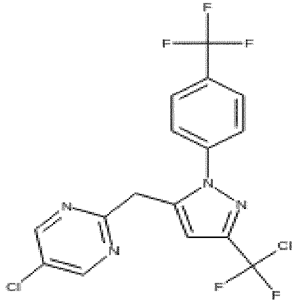 | 8.64 (s, 2H), 7.82 - 7.68 (m, 4H), 6.60 (s, 1H), 4.37 (s, 2H) |
| 1.037 | 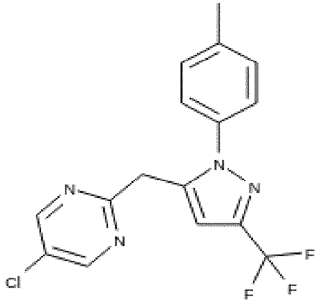 | 8.62 (s, 2H), 7.39 - 7.34 (m, 2H), 7.28 - 7.23 (m, 2H), 6.53 (s, 1H), 4.32 (s, 2H), 2.41 (s, 3H) |
| 1.039 | 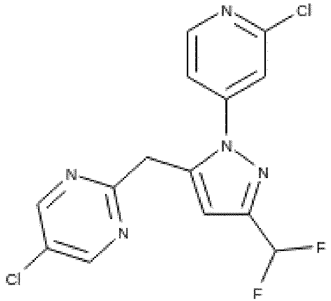 | 8.66 (s, 2 H) 8.50 (d, 1 H) 7.75 (d, 1 H) 7.61 (dd, 1 H) 6.61 (s, 1 H) 6.52 - 6.89 (m, 1 H) 4.48 (s, 2 H) |
| 1.040 | 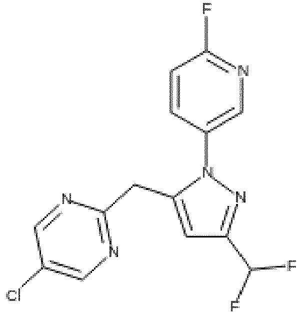 | 8.63 (s, 2H), 8.48 (dd, 1H), 8.05 (ddd, 1H), 7.08 (dd, 1H), 6.70 (t, 1H), 6.58 (s, 1H), 4.33 (s, 2H) |
| 1.041 | 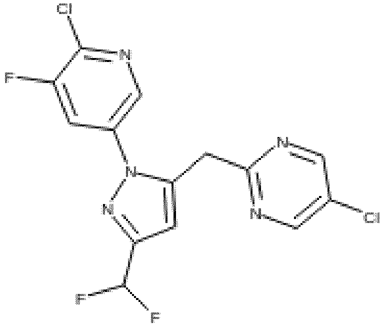 | 8.65 (s, 2H), 8.59 (d, 1H), 7.95 (dd, 1H), 6.69 (t, 1H), 6.61 (s, 1H), 4.38 (s, 2H) |
| 1.042 | 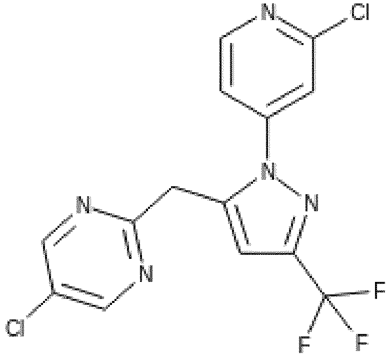 | 8.67 (s, 2 H) 8.52 (d, 1 H) 7.77 (d, 1 H) 7.63 (dd, 1 H) 6.66 (s, 1 H) 4.48 (s, 2 H) |
| 1.043 | 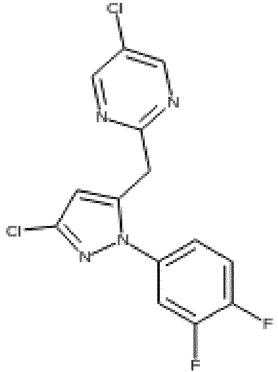 | 8.64 (s, 2H), 7.48 (ddd, 1H), 7.34 - 7.29 (m, 1H), 7.28 - 7.20 (m, 1H), 6.25 (s, 1H), 4.30 (s, 2H) |
| 1.044 | 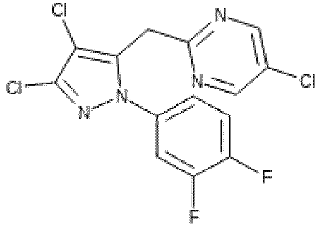 | 8.63 (s, 2H), 7.46 (ddd, 1H), 7.31 - 7.17 (m, 2H), 4.36 (s, 2H) |
| 1.045 | 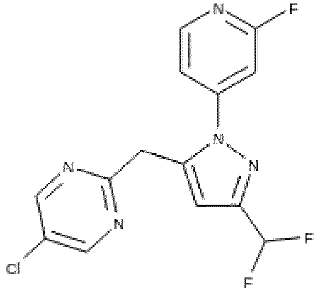 | 8.33 (d, 1 H) 7.57 (dt, 1 H) 7.37 (t, 1 H) 6.61 (s, 1 H) 6.52 - 6.90 (m, 1 H) 4.50 (s, 2 H) |
| 1.046 | 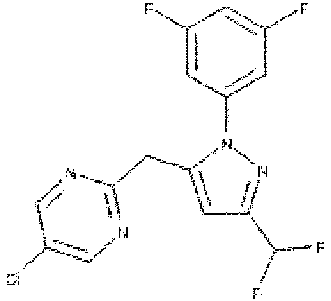 | 8.65 (s, 2H), 7.25 - 7.18 (m, 2H), 6.90 (tt, 1H), 6.69 (t, 1H), 6.54 (s, 1H), 4.40 (s, 2H) |
| 1.047 | 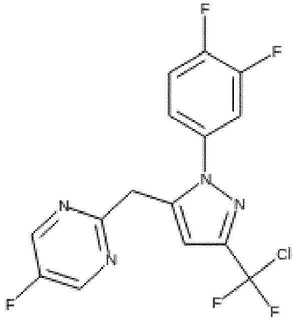 | 8.55 (s, 2 H), 7.47 - 7.54 (m, 1 H), 7.32 - 7.38 (m, 1 H), 7.24 - 7.30 (m, 1 H), 6.55 (s, 1 H), 4.34 (s, 2 H) |
| 1.048 | 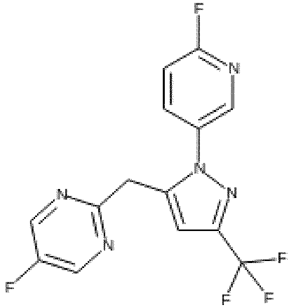 | 8.53 (s, 2 H), 8.49 (d, 1 H), 8.06 (ddd, 1 H), 7.08 (dd, 1 H), 6.62 (s, 1 H), 4.35 (s, 2 H) |

### Biological Examples

Seeds of a variety of test species are sown in standard soil in pots *Amaranthus palmeri* (AMAPA), *Amaranthus retoflexus* (AMARE), *Setaria faberi* (SETFA), *Echinochloa crus-galli* (ECHCG), *Ipomoea hederacea* (IPOHE)). After cultivation for one day (pre-emergence) or after 8 days cultivation (post-emergence) under controlled conditions in a glasshouse (at 24/16°C, day/night; 14 hours light; 65% humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 250 g/ha unless otherwise stated. The test plants are then grown in a glasshouse under controlled conditions in a glasshouse (at 24/16°C, day/night; 14 hours light; 65% humidity) and watered twice daily. After 13 days for pre and post-emergence, the test is evaluated for the percentage damage caused to the plant. The biological activities are shown in the following table on a five-point scale (5 = 81-100%; 4 = 61-80%; 3=41-60%; 2=21-40%; 1=0-20%).

**TABLE B1. Post-emergence Test**

| Compound | Rate (g/ha) | AMAPA | AMARE | SETFA | ECHCG | IPOHE |
|---|---|---|---|---|---|---|
| 1.001 | 250 | 4 | 4 | 4 | 4 | 4 |
| 1.002 | 250 | 4 | 4 | 4 | 5 | 4 |
| 1.003 | 250 | 5 | 5 | 4 | 5 | 5 |
| 1.004 | 250 | 5 | 5 | 5 | 5 | 4 |
| 1.007 | 250 | 5 | 5 | 5 | 5 | 3 |
| 1.008 | 250 | 5 | 5 | 5 | 5 | 3 |
| 1.009 | 250 | 5 | 5 | - | 5 | 4 |
| 1.011 | 250 | 5 | 5 | 5 | 5 | 3 |
| 1.012 | 250 | 5 | 5 | 5 | 4 | 4 |
| 1.016 | 250 | 5 | 5 | 5 | 5 | 3 |
| 1.019 | 250 | 5 | 5 | - | 4 | 3 |
| 1.020 | 250 | 5 | 5 | - | 5 | 4 |
| 1.021 | 250 | 5 | 5 | - | 5 | 4 |
| 1.022 | 250 | 5 | 5 | - | 5 | 3 |
| 1.023 | 250 | 5 | 5 | - | 4 | 3 |
| 1.024 | 250 | 5 | 5 | - | 5 | 4 |
| 1.025 | 250 | - | 5 | 5 | 5 | 4 |
| 1.026 | 250 | - | 5 | 5 | 5 | 2 |
| 1.027 | 250 | 5 | 5 | - | 5 | 5 |
| 1.028 | 250 | 5 | 5 | - | 5 | 4 |
| 1.029 | 250 | 5 | 5 | - | 4 | 4 |
| 1.030 | 250 | 5 | 5 | - | 4 | 4 |
| 1.031 | 250 | 4 | 5 | - | 4 | 4 |
| 1.032 | 250 | 5 | 5 | - | 4 | 4 |
| 1.033 | 250 | 5 | 5 | - | 4 | 4 |
| 1.034 | 250 | 5 | 4 | - | 4 | 4 |
| 1.035 | 250 | 4 | 4 | 3 | 3 | 4 |
| 1.036 | 250 | - | 5 | 5 | 5 | 3 |
| 1.037 | 250 | - | 5 | 5 | 4 | 3 |

**TABLE B2. Pre-emergence Test**

| Compound | Rate (g/ha) | AMAPA | AMARE | SETFA | ECHCG | IPOHE |
|---|---|---|---|---|---|---|
| 1.001 | 250 | 5 | 5 | 5 | 5 | 5 |
| 1.002 | 250 | 5 | 5 | 4 | 5 | 5 |
| 1.003 | 250 | 5 | 5 | 5 | 5 | 5 |
| 1.004 | 250 | 5 | 5 | 5 | 5 | 5 |
| 1.007 | 250 | 5 | 5 | 5 | 5 | 5 |
| 1.008 | 250 | 5 | 5 | 5 | 5 | 4 |
| 1.009 | 250 | 5 | 5 | - | 5 | 5 |
| 1.011 | 250 | 5 | 5 | 5 | 5 | 5 |
| 1.012 | 250 | 5 | 5 | 5 | 5 | 5 |
| 1.016 | 250 | 5 | 5 | 5 | 5 | 5 |
| 1.019 | 250 | 5 | 5 | - | 4 | 5 |
| 1.020 | 250 | 5 | 5 | - | 5 | 5 |
| 1.021 | 250 | 5 | 5 | - | 5 | 5 |
| 1.022 | 250 | 5 | 5 | - | 5 | 5 |
| 1.023 | 250 | 5 | 5 | - | 5 | 5 |
| 1.024 | 250 | 5 | 5 | - | 5 | 5 |
| 1.025 | 250 | 5 | 5 | - | 5 | 5 |
| 1.026 | 250 | 5 | 5 | - | 5 | 5 |
| 1.027 | 250 | 5 | 5 | - | 5 | 5 |
| 1.028 | 250 | 5 | 5 | - | 5 | 5 |
| 1.029 | 250 | 5 | 5 | - | 5 | 5 |
| 1.030 | 250 | 5 | 5 | - | 5 | 4 |
| 1.031 | 250 | 5 | 5 | - | 5 | 5 |
| 1.032 | 250 | 5 | 5 | - | 5 | 5 |
| 1.033 | 250 | 5 | 5 | - | 5 | 5 |
| 1.034 | 250 | 5 | 5 | - | 5 | 5 |
| 1.035 | 250 | 5 | 5 | 5 | 4 | 4 |
| 1.036 | 250 | 5 | 5 | 5 | 5 | 5 |
| 1.037 | 250 | 5 | 5 | 5 | 5 | 1 |

## Claims

1. A compound of Formula (I): 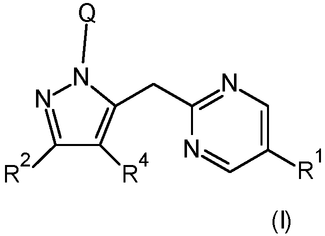
or an agronomically acceptable salt thereof,
wherein
Q is selected from the group consisting of phenyl, a C-linked 6-membered heteroaryl, Q-A and Q-B 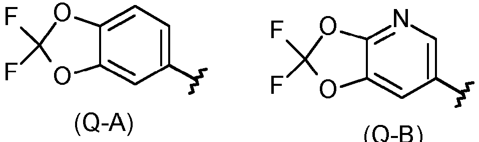
wherein said phenyl or 6-membered heteroaryl are optionally substituted by one or more independent R³ substituents;
R¹ is independently selected from the group consisting of halogen, -CN, NO₂, C₁-C₄alkyl, C₁-C₄haloalkyl, C₃-C₆cycloalkyl, C₂-C₄alkenyl, C₂-C₄alkynyl, - S(O)ₚC₁-C₄alkyl, C₁-C₄alkoxy-, -C(O)C₁-C₄alkyl, -C(O)OC₁-C₄alkyl, C₁-C₄haloalkoxy and C₁-C₄alkoxyC₁-C₃alkyl-;
R² is selected from the group consisting of halogen, -CN, C₁-C₂alkyl and C₁-C₂haloalkyl;
R³ is selected from the group consisting of halogen, C₁-C₄ alkyl, C₁-C₄ haloalkyl, C₁-C₄ alkoxy, C₁-C₄ haloalkoxy, -CN, NO₂, C₂-C₄alkenyl, C₂-C₄alkynyl, -S(O)ₚC₁-C₄alkyl;
R⁴ is selected from the group consisting of hydrogen, halogen, -CN, C₁-C₂alkyl and C₁-C₂haloalkyl; and
p = 0, 1 or 2.

2. A compound of Formula (I) according to claim 1, wherein Q is selected from the group consisting of:

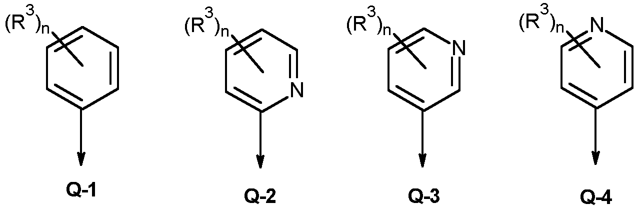

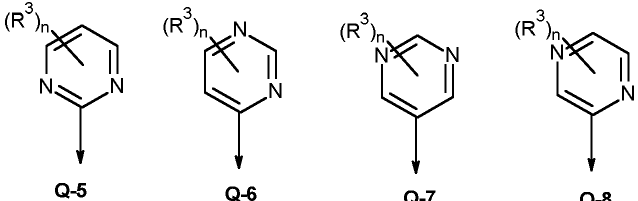

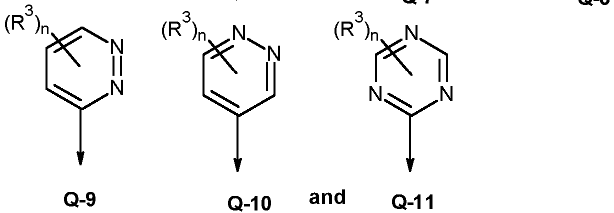

3. A compound according to claim 2, wherein Q is Q-1 or Q-3.

4. A compound according to claim 2 or claim 3, wherein n is 1.

5. A compound according to claim 4, wherein R³ is halogen or -CF₃.

6. A compound according to any one of the previous claims, wherein Q is 4-CF₃-phenyl-.

7. A compound according to any one of the previous claims, wherein R¹ is selected from the group consisting of halogen, CN, C₁-C₄alkyl, C₁-C₄haloalkyl, C₁-C₄alkoxy and C₁-C₄haloalkoxy.

8. A compound according to claim 7, wherein R¹ is selected from the group consisting of methyl, fluoro, chloro, bromo and methoxy-.

9. A compound according to claim 8, wherein R¹ is chloro.

10. A compound according to any one of the previous claims, wherein R² is -CF₃ or -CF₂H.

11. A compound according to any one of the previous claims, wherein R⁴ is hydrogen.

12. A herbicidal composition comprising a compound according to any one of the previous claims and an agriculturally acceptable formulation adjuvant.

13. A herbicidal composition according to claim 12, further comprising at least one additional pesticide.

14. A herbicidal composition according to claim 13, wherein the additional pesticide is a herbicide or herbicide safener.

15. A method of controlling weeds at a locus comprising application to the locus of a weed controlling amount of a composition according to any one of claims 12 to 14.

16. Use of a compound of Formula (I) as defined in claim 1 as a herbicide.

## Patentansprüche

1. Verbindung der Formel (I): 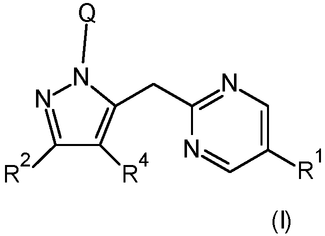
oder ein agronomisch unbedenkliches Salz davon,
wobei
Q aus der Gruppe bestehend aus Phenyl, einem C-verknüpften 6-gliedrigen Heteroaryl, Q-A und Q-B ausgewählt ist, 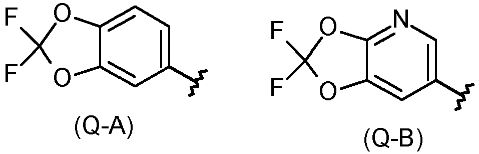
wobei das Phenyl oder 6-gliedrige Heteroaryl gegebenenfalls durch einen oder mehrere unabhängige R³-Substituenten substituiert ist;
R¹ unabhängig aus der Gruppe bestehend aus Halogen, -CN, NO₂, C₁-C₄-Alkyl, C₁-C₄-Halogenalkyl, C₃-C₆-Cycloalkyl, C₂-C₄-Alkenyl, C₂-C₄-Alkinyl, -S(O)ₚC₁-C₄-Alkyl, C₁-C₄-Alkoxy, -C(O)C₁-C₄-Alkyl, -C(O)OC₁-C₄-Alkyl, C₁-C₄-Halogenalkoxy und C₁-C₄-Alkoxy-C₁-C₃-alkyl ausgewählt ist;
R² aus der Gruppe bestehend aus Halogen, -CN, C₁-C₂-Alkyl und C₁-C₂-Halogenalkyl ausgewählt ist;
R³ aus der Gruppe bestehend aus Halogen, C₁-C₄-Alkyl, C₁-C₄-Halogenalkyl, C₁-C₄-Alkoxy, C₁-C₄-Halogenalkoxy, -CN, NO₂, C₂-C₄-Alkenyl, C₂-C₄-Alkinyl, -S(O)ₚC₁-C₄-Alkyl ausgewählt ist;
R⁴ aus der Gruppe bestehend aus Wasserstoff, Halogen, - CN, C₁-C₂-Alkyl und C₁-C₂-Halogenalkyl ausgewählt ist; und p = 0, 1 oder 2.

2. Verbindung der Formel (I) nach Anspruch 1, wobei Q ausgewählt ist aus der Gruppe bestehend aus:

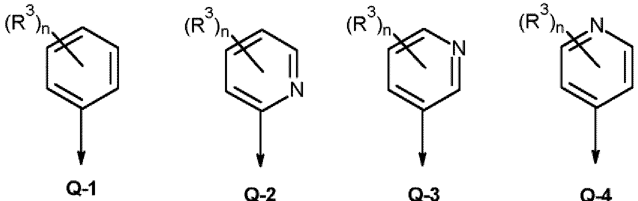

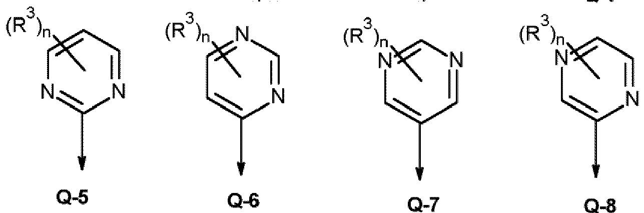

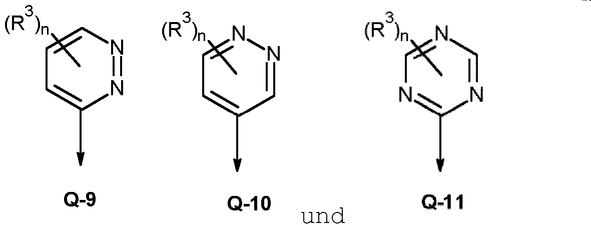

3. Verbindung nach Anspruch 2, wobei Q für Q-1 oder Q-3 steht.

4. Verbindung nach Anspruch 2 oder Anspruch 3, wobei n für 1 steht.

5. Verbindung nach Anspruch 4, wobei R³ für Halogen oder -CF₃ steht.

6. Verbindung nach einem der vorhergehenden Ansprüche, wobei Q für 4-CF₃-Phenyl- steht.

7. Verbindung nach einem der vorhergehenden Ansprüche, wobei R¹ aus der Gruppe bestehend aus Halogen, CN, C₁-C₄-Alkyl, C₁-C₄-Halogenalkyl, C₁-C₄-Alkoxy und C₁-C₄-Halogenalkoxy ausgewählt ist.

8. Verbindung nach Anspruch 7, wobei R¹ aus der Gruppe bestehend aus Methyl, Fluor, Chlor, Brom und Methoxyausgewählt ist.

9. Verbindung nach Anspruch 8, wobei R¹ für Chlor steht.

10. Verbindung nach einem der vorhergehenden Ansprüche, wobei R² für -CF₃ oder -CF₂H steht.

11. Verbindung nach einem der vorhergehenden Ansprüche, wobei R⁴ für Wasserstoff steht.

12. Herbizide Zusammensetzung, umfassend eine Verbindung nach einem der vorhergehenden Ansprüche und ein landwirtschaftlich unbedenkliches Formulierungshilfsmittel.

13. Herbizide Zusammensetzung nach Anspruch 12, ferner umfassend mindestens ein zusätzliches Pestizid.

14. Herbizide Zusammensetzung nach Anspruch 13, wobei es sich bei dem zusätzlichen Pestizid um ein Herbizid oder einen Herbizid-Safener handelt.

15. Verfahren zur Bekämpfung von Unkräutern an einem Standort, bei dem man eine unkrautbekämpfende Menge einer Zusammensetzung nach einem der Ansprüche 12 bis 14 auf den Standort ausbringt.

16. Verwendung einer Verbindung der Formel (I) gemäß Anspruch 1 als Herbizid.

## Revendications

1. Composé de formule (I) : 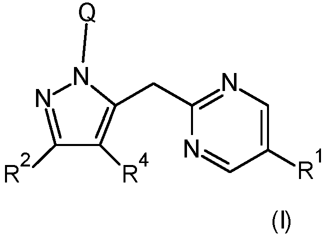 ou sel acceptable sur le plan agronomique correspondant, Q étant choisi dans le groupe constitué par phényle, un hétéroaryle à 6 chaînons C-lié, Q-A et Q-B 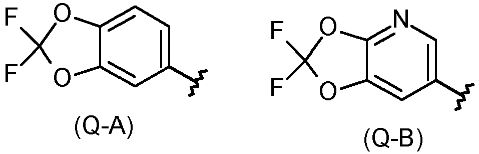
lesdits phényle or hétéroaryle à 6 chaînons étant éventuellement substitués par un ou plusieurs substituants R³ indépendants ;
R¹ étant indépendamment choisi dans le groupe constitué par halogène, -CN, NO₂, C₁-C₄alkyle, C₁-C₄halogénoalkyle, C₃-C₆cycloalkyle, C₂-C₄alcényle, C₂-C₄alcynyle, -S(O)ₚC₁-C₄alkyle, C₁-C₄alcoxy-, -C(O)C₁-C₄alkyle, -C(O)OC₁-C₄alkyle, C₁-C₄halogénoalcoxy et C₁-C₄alcoxyC₁-C₃alkyle- ; R² étant choisi dans le groupe constitué par halogène, - CN, C₁-C₂alkyle et C₁-C₂halogénoalkyle ;
R³ étant choisi dans le groupe constitué par halogène, C₁-C₄ alkyle, C₁-C₄ halogénoalkyle, C₁-C₄ alcoxy, C₁-C₄ halogénoalcoxy, -CN, NO₂, C₂-C₄alcényle, C₂-C₄alcynyle, - S(O)ₚC₁-C₄alkyle ;
R⁴ étant choisi dans le groupe constitué par hydrogène, halogène, -CN, C₁-C₂alkyle et C₁-C₂halogénoalkyle ; et
p = 0, 1 ou 2.

2. Composé de formule (I) selon la revendication 1, Q étant choisi dans le groupe constitué par :

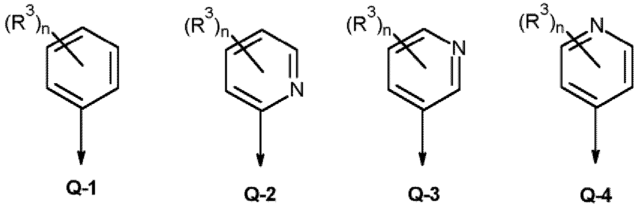

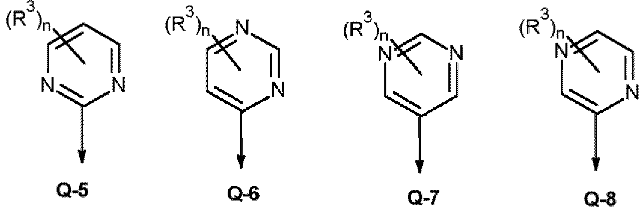

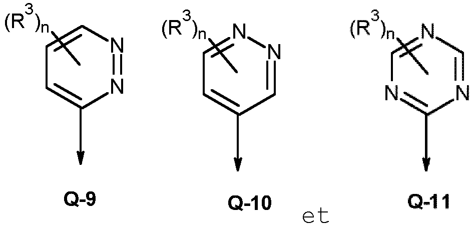

3. Composé selon la revendication 2, Q étant Q-1 ou Q-3.

4. Composé selon la revendication 2 ou la revendication 3, n étant 1.

5. Composé selon la revendication 4, R³ étant halogène ou -CF₃.

6. Composé selon l'une quelconque des revendications précédentes, Q étant 4-CF₃-phényle-.

7. Composé selon l'une quelconque des revendications précédentes, R¹ étant choisi dans le groupe constitué par halogène, CN, C₁-C₄alkyle, C₁-C₄halogénoalkyle, C₁-C₄alcoxy et C₁-C₄halogénoalcoxy.

8. Composé selon la revendication 7, R¹ étant choisi dans le groupe constitué par méthyle, fluoro, chloro, bromo et méthoxy-.

9. Composé selon la revendication 8, R¹ étant chloro.

10. Composé selon l'une quelconque des revendications précédentes, R² étant -CF₃ ou -CF₂H.

11. Composé selon l'une quelconque des revendications précédentes, R⁴ étant hydrogène.

12. Composition herbicide comprenant un composé selon l'une quelconque des revendications précédentes et un adjuvant de formulation acceptable sur le plan agricole.

13. Composition herbicide selon la revendication 12, comprenant en outre au moins un pesticide supplémentaire.

14. Composition herbicide selon la revendication 13, le pesticide supplémentaire étant un herbicide ou un phytoprotecteur herbicide.

15. Procédé de lutte contre des adventices au niveau d'un lieu comprenant une application sur le lieu d'une quantité de lutte contre des adventices d'une composition selon l'une quelconque des revendications 12 à 14.

16. Utilisation d'un composé de formule (I) tel que défini dans la revendication 1 comme un herbicide.
